(19) Europäisches Patentamt
European Patent Office
Office européen des brevets

(11) Veröffentlichungsnummer: **0 362 622 B1**

# (12) EUROPÄISCHE PATENTSCHRIFT

(45) Veröffentlichungstag der Patentschrift: **29.03.95**

(51) Int. Cl.6: **A61K 31/71**, C07D 503/00, //(A61K31/71,31:42)

(21) Anmeldenummer: **89117433.6**

(22) Anmeldetag: **21.09.89**

## (54) Verwendung stabiler Oxapenem-3-carbonsäuren zur Herstellung Beta-Lactamase hemmender Arzneimittel.

(30) Priorität: **04.10.88 DE 3833693**

(43) Veröffentlichungstag der Anmeldung: **11.04.90 Patentblatt 90/15**

(45) Bekanntmachung des Hinweises auf die Patenterteilung: **29.03.95 Patentblatt 95/13**

(84) Benannte Vertragsstaaten: **AT BE CH DE ES FR GB GR IT LI LU NL SE**

(56) Entgegenhaltungen: **EP-A- 0 301 394**

(73) Patentinhaber: **ELI LILLY AND COMPANY**
**Lilly Corporate Center**
**Indianapolis**
**Indiana 46285 (US)**

(72) Erfinder: **Pfaendler, Hans-Rudolf, Prof. Dr.**
**Pippinplatz 1**
**D-8000 München 71 (DE)**
Erfinder: **Metzger, Karl-Georg, Dr.**
**Pahlkestrasse 75**
**D-5600 Wuppertal 1 (DE)**
Erfinder: **Endermann, Rainer, Dr.**
**In den Birken 152a**
**D-5600 Wuppertal 1 (DE)**
Erfinder: **Haller, Ingo, Dr.**
**Dornröschenweg 4**
**D-5600 Wuppertal 1 (DE)**
Erfinder: **Wild, Hanno, Dr.**
**Am Wolfshahn 19**
**D-5600 Wuppertal 1 (DE)**
Erfinder: **Hartwig, Wolfgang, Dr.**
**Pahlkestrasse 3**
**D-5600 Wuppertal 1 (DE)**

(74) Vertreter: **Hudson, Christopher Mark et al**
**Lilly Industries Limited**
**European Patent Operations**
**Erl Wood Manor**
**Windlesham**
**Surrey GU20 6PH (GB)**

EP 0 362 622 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung von 1-Oxapenem-3-carbonsäuren der folgenden Strukturen

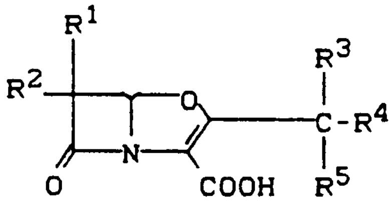

bzw.

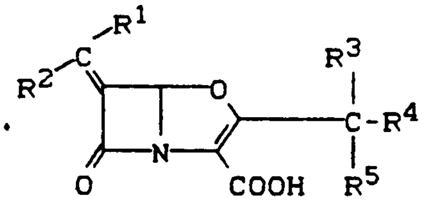

in denen

$R^1$ und $R^2$ unabhängig voneinander Wasserstoff oder via Kohlenstoffatome gebundene pharmazeutisch annehmbare Gruppen bedeuten und $R^3$, $R^4$ und $R^5$ unabängig voneinander pharmazeutisch annehmbare Gruppen bedeuten, die via Kohlenstoffatome an das exocyclische, allylische Kohlenstoffatom gebunden sind,

zur Herstellung $\beta$-Lactamase-hemmender Arzneimittel.

Die Verbindungen wie auch ihre pharmazeutisch annehmbaren Salze, Ester und Amidderivate sind auch nützliche Antibiotika und als solche aus dem 54(3)-Dokument EP-A-0301394 bekannt.

Die erfindungsgemäß verwendeten Verbindungen sind wirksame ß-Lactamasehemmer. ß-Lactamasen sind Enzyme, die von vielen pathogenen, klinisch relevanten Bakterien gebildet werden, so daß sie von herkömmlichen Antibiotika nicht mehr wirksam gehemmt werden. Um Infektionen mit solchen Bakterien zu bekämpfen, werden in den Kliniken pharmazeutische Zubereitungen verabreicht, die neben einem herkömmlichen Antibiotikum einen ß-Lactamasehemmer in der Regel in einem 1:1-Verhältnis enthalten. Beispiele finden sich z.B. in Chemical and Engineering News 64, (39), Seiten 33 bis 67 (1986).

Diese Art von ß-Lactamasehemmern ist für sich antibakteriell nicht oder nur wenig wirksam; ihre Aufgabe ist der Schutz eines herkömmlichen Antibiotikums vor den angreifenden bakteriellen ß-Lactamasen.

Die Wirkung als ß-Lactamasehemmer von früher hergestellten Oxapenem-3-carbonsäuren war wegen ihrer geringen hydrolytischen Stabilität nur an zellfreien Enzymen festzustellen. Beispielsweise war das Kaliumsalz von 2-Ethyl-1-oxapenem-3-carbonsäure zu instabil für die Prüfung des Synergismus mit Ampicillin gegenüber intakten Bakterien (Chemistry and Biology of ß-Lactam Antibiotics Vol. 2, Nontraditional ß-Lactam Antibiotics, ed. by R.B. Morin and M. Gorman, Academic Press, New York, Seite 383 (1982)).

Im Gegensatz zu diesen früher hergestellten, instabilen Oxapenem-3-carbonsäuren sind die stabilen erfindungsgemäß verwendeten Verbindungen auch in der Gegenwart von intakten Bakterien als ß-Lactamasehemmer voll wirksam.

Ein besonderer Vorteil der erfindungsgemäß verwendeten Oxapenem-3-carbonsäurenist, daß sie neben den oben erwähnten Eigenschaften der ß-Lactamasehemmung auch für sich selber antibakteriell wirksam sind. Zwar sind Stoffe mit ähnlichen Eigenschaften bekannt, z.B. Formimino-thienamycin (Lit.: Recent Advances in the Chemistry of ß-Lactam Antibiotics, ed. by G.I. Gregory, The Royal Society, London, Seite 279 (1981)). Die erfindungsgemäß verwendeten Verbindungen zeigen jedoch eine raschere, progressivere und irreversiblere Hemmung der ß-Lactamasen bei vielen klinisch relevanten Bakterien. So wird beispielsweise die Cephalosporinase von E. cloacae 908 R in vitro 780 mal wirksamer gehemmt vom erfindungsgemäßen 2-tert.-Butyl-6-hydroxymethyloxapenem-3-carbonsäure-K-Salz als von Formimino-thienamycin.

Die erfindungsgemäß verwendeten Oxapenem-3-carbonsäuren können im Verhältnis 1:1 mit einem herkömmlichen Antibiotikum kombiniert werden. Die hohe Wirksamkeit als ß-Lactamasehemmer erlaubt es aber, Kombinationen mit weit geringeren Anteilen der erfindungsgemäßen Stoffe herzustellen. So ist in den meisten Fällen ein Verhältnis von 1:10 zwischen den erfindungsgemäß verwendeten Stoffen und dem herkömmlichen Antibiotikum ausreichend, um das Wachstum de ß-lactamasebildenden Keime in vitro rasch und wirkungsvoll zu hemmen. Aber auch andere Verhältnisse von 1:50 bis 1:1 sind möglich. Die erfindungsgemäß verwendeten Oxapenem-3-carbonsäuren können als Racemat oder in der (5R)-enantiomerenreinen Form in den obengenannten Kombinationen verwendet werden.

Herkömmliche Antibiotika, die in pharmazeutischen Zubereitungen gemeinsam mit den erfindungsgemäß verwendeten Oxapenem-3-carbonsäuren verwendet werden können, sind klassische (wie z.B. die Penicilline und Cephalosporine) wie auch nichtklassische (wie z.B. Peneme, Catapeneme oder Monobactame) ß-Lactamantibiotika. Beispiele dafür sind Ampicillin, Amoxicillin, Azlocillin, Mezlocillin, Ticarcillin,

Cefoperazon, Cephalexin, Cefudor, Cefaloridin, Cefazolin, Ceftrazidin, Methicillin, Mecillinam, Penicillin G, Azthreonam, Formimino-thienamycin, Moxalactam u.ä.

Solche Zubereitungen sind effektiv gegen gram-positive, gram-negative, aerobe und anaerobe ß-lactamasebildende und sensitive Bakterien, wie z.B. Staphylokokken, Streptokokken und Enterobakterien. Auch eigentliche Problemkeime, wie z.B. ß-lactamasebildende Pseudomonas aeruginosa werden effektiv gehemmt von bestimmten Zubereitungen. Entscheidend ist hier, daß das herkömmliche Antibiotikum, mit dem die erfindungsgemäß verwendete Oxapenem-3-carbonsäure kombiniert wird, in das Innere des Bakteriums einzudringen vermag.

Bevorzugt sind phamazeutische Zubereitungen von Penicillinen und Cephalosporinen mit den erfindungsgemäß verwendeten Oxapenem-3-carbonsäuren; besonders bevorzugt sind Kombinationen von Penicillinen mit 2-tert.-Butyl-6-hydroxy-methyloxapenem-3-carbonsäure-K-Salz.

Die Erfindung betrifft die Verwendung von 6-unsubstituierten, 6-mono oder 6,6-disubstituierten 1-Oxapen-2-em-3-carbonsäuren, die in 2-Stellung mit besonderen Resten versehen sind. Diese Rest sind dadurch gekennzeichnet, daß sie ein zentrales Kohlenstoffatom besitzen, welches direkt an den Oxapenem-Nucleus gebunden ist und welches drei weitere, über C-Atome verbundene Gruppen gebunden hält. Diese Verbindungen sind nützliche Antibiotika und sie können durch die allgemeinen Strukturformeln.

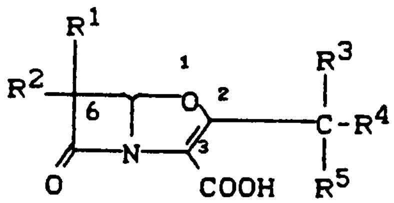

bzw.

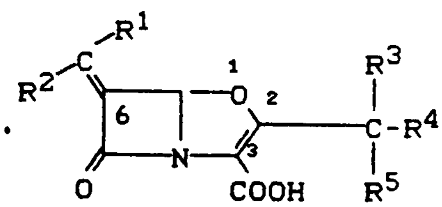

dargestellt werden, in denen $R^1$ und $R^2$ unabhängig voneinander ausgewählt werden aus: Wasserstoff oder den pharmazeutisch annehmbaren, über C-C-Einfachbindungen an den übrigen Molekülteil gebundenen Gruppen, die enthalten: substituiertes oder unsubstituiertes Alkyl, Alkenyl, Alkinyl, Cycloalkyl, Alkylcycloalkyl, Alkylcycloalkenyl, Cycloalkylalkyl, Alkenylcycloalkyl, Cycloalkenylalkyl, Aryl, Aralkyl, Aralkenyl, Aralkinyl, Carboxy oder Cyano, worin die vorhergehenden Alkyl-, Alkenyl- oder Alkinyl-Molekülteile 1 bis 6 Kohlenstoffatome, die Cycloalkyl- oder die Cycloalkenyl-Molekülteile 3 bis 6 und die Aryl-Molekülteile 6 bis 10 Kohlenstoffatome enthalten, Heteroaryl, Heteroaralkyl, Heteroaralkenyl, Heteroaralkinyl, Alkylheteroaryl, Heterocyclyl, Heterocyclylakyl, Heterocyclylalkenyl, Heterocyclylalkinyl, Alkylheterocyclyl, worin die vorhergehenden Alkyl-, Alkenyl- oder die Alkinyl-Molekülteile 1 bis 6 Kohlenstoffatome enthalten und der heteroaromatische oder heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und wobei die Substituenten der oben aufgeführten Gruppen sein können; Geschütztes oder ungeschütztes Hydroxy, Hydroxyalkyloxy, Aminoalkyloxy, Amidinoalkyloxy, Alkyloxy, Acyloxy, Aryloxy, Heteroaryloxy, Heterocyclyloxy, Carbamoyl, Carbamoyloxy, Thiocarbamoyl, Thiocarbamoyloxy, Alkylcarbamoyloxy, Alkylthiocarbamoyloxy, Mercapto, Alkylthio, Hydroxyalkylthio, Aminoalkylthio, Amidinoalkylthio, Acylthio, Arylthio, Alkylheteroarylthio, Hydroxyalkylheteroarylthio, Heterocyclylthio, Carbamoylthio, Alkylcarbamoylthio, Thiocarbamoylthio, Alkylthiocarbamoylthio, geschütztes oder ungeschütztes Amino oder Monoalkylamino, Dialkylamino, Oxo, geschütztes oder ungeschütztes Oximino oder Alkylimino, Tetraalkylammonium, Cycloalkylamino, Arylamino, Heteroarylamino, Heterocyclylamino, Acylamino, Amidino, Alkylamidino, Guanidino, Alkylguanidino, Carbamoylamino, Alkylcarbamoylamino, Thiocarbamoylamino, Alkylthiocarbamoylamino, Nitro, Chlor, Brom, Fluor, Iod, Azido, Cyano, Alkylsulfinyl, Alkylsulfonyl, Sulfonamido, Sulfamoyloxy, Alkylsulfonyloxy oder geschütztes oder ungeschütztes Sulfo, Sulfoxy oder Carboxy, wobei die Substituenten unabhängig voneinander ein- oder mehrfach auftreten und ihr Alkyl-Molekülteil 1 bis 6 Kohlenstoffatome, ihr Aryl-Molekülteil 6 bis 10 Kohlenstoffatome enthält, und wobei der heteroaromatische oder heterocyclische Molekülteil mono-oder bicyclisch ist und 3 bis 10 Ringatome enthält, wovon eines oder mehrere ausgewählt werden aus der Gruppe: Sauerstoff, Schwefel und Stickstoff, und dadurch gekennzeichnet, daß $R^3$, $R^4$ und $R^5$ unabhängig voneinander ausgewählt werden aus den vorhergehend genannten, über Kohlenstoff-Kohlenstoff-Einfachbindungen an den übrigen Molekülteil gebundenen pharmazeutisch annehmbaren Gruppen.

Die Gruppen $R^3$, $R^4$ und $R^5$ werden unabhängig ausgewählt aus den pharmazeutisch annehmbaren, über C-C-Einfachbindungen an den übrigen Molekülteil gebundenen Gruppen, wie sie obenstehend beschrieben sind.

Die Schutzgruppen der oben erwähnten, geschützten Substituenten sind an sich bekannte, leicht entfernbare Reste, wie sie üblicherweise in der organischen Synthese zu diesem Zweck Verwendung finden. Solche Schutzgruppen finden sich beispielsweise in T.W.Greene, "Protective Groups in Organic Synthesis", Wiley, New York, 1981.

Weiterhin können zwei der Gruppen $R^3$, $R^4$ oder $R^5$ über Kohlenstoff, Sauerstoff, Stickstoff und Schwefel enthaltende Molekülteile miteinander verbrückt sein; sie sind dann Bestandteil eines carbocyclischen oder heterocyclischen Ringes, der drei-, vier-, fünf- oder sechsgliedrig sein kann.

Weiterhin können die beiden Gruppen $R^1$ und $R^2$ über Kohlenstoff, Sauerstoff, Stickstoff und Schwefel enthaltende Molekülteile miteinander verbrückt sein; sie sind dann Bestandteil eines drei-, vier-, fünf- oder sechsgliedrigen carbo- oder heterocyclischen Ringes.

Beispiele für verbrückende Molekülteile für $R^1$ und $R^2$ bzw. für $R^3$ und $R^4$ sind Methylen, Dimethylen, Trimethylen, Tetramethylen, Oxamethylen, Oxadimethylen, Dioxamethylen, Azadimethylen, Diazamethylen o.ä.

Pharmazeutisch annehmbare Gruppen $R^1$, $R^2$, $R^3$, $R^4$ und $R^5$, die über C-C-Einfachbindung gebunden sind, sind Gruppen, wie sie beispielsweise bei den $\beta$-Lactamantibiotika üblich sind. Solche Gruppen findet man z.B. in M.L. Sassiver, A. Lewis in "Advances in Applied Microbiology, ed. D. Perlman, Academic Press, N.Y. (1970).

Die Erfindung betrifft weiterhin die Verwendung der pharmazeutisch annehmbaren Salze, Ester und Amidderivate der Verbindungen (I) und (II).

Neben den klassischen $\beta$-Lactamantibiotika, d.h. den Penicillinen und Cephalosporinen werden heute auch die sogenannten nichtklassischen oder nichttraditionellen $\beta$-Lactamantibiotika gegen bakterielle Infektionskrankheiten eingesetzt. Die wichtigsten heute angewandten Verbindungen dieses Typs sind die Peneme und die Carbapeneme. Ein kürzlich erschienenes Buch handelt die Synthese und Pharmakologie dieser neuen Wirkstoffe: Chemistry and Biology of $\beta$-Lactam Antibiotics, Vol. 2 (Nontraditional ß-Lactam Antibiotics), ed. by R.B. Morin and M. Gorman, Academic Press, New York (1982).

Auf Grund der engen strukturellen Verwandtschaft der Oxapenemcarbonsäuren mit den schwefelhaltigen Penemcarbonsäuren oder mit den Carbapenemcarbonsäuren konnte vermutet werden, daß Oxapenem-3-carbonsäuren auch antibakteriell wirksam seien (Tetrahedron 38 (16) 2489-2504 (1982), Seite 2489).

Obwohl eine antibakterielle Wirksamkeit von Oxapenem-3-carbonsäuren erwähnt wurde, z.B. in US 4, 172, 895 oder EP 0 018 305 A1 ist sie niemals durch experimentelle Daten belegt worden. Die einzigen verfügbaren Meßdaten über deren antibakterielle Wirksamkeit finden sich in "Chemistry and Biology of ß-Lactam Antibiotics, Vol. 2 Nontraditional ß-Lactam Antibiotics" ed. by R.B. Morin and M. Gorman, Seite 383: "(Das Kaliumsalz von 2-Ethyl-1-oxapenem-3-carbonsäure) war zu instabil für die Prüfung der antibakteriellen Aktivität oder des Synergismus mit Ampicillin gegenüber intakten Bakterien."

Eine in früheren Patentanmeldungen als wirksam vorgestellte Verbindung, die 2-Ethyl-1-oxapen-2-em-3-carbonsäure, war also in Wirklichkeit viel zu wenig stabil in wäßrigem Medium für eine antibakterielle Prüfung und damit als Antibiotikum praktisch unwirksam. Lediglich eine Hemmung von isolierten bakteriellen Enzymen ($\beta$-Laktamasen) war nachweisbar.

Die Instabilität früher bekanntgewordener Oxapenem-3-carbonsäuren, auch Clavem-carbonsäuren genannt, äußerte sich auch bei der Herstellung der Methylester z.B. in J.C.S. Chem. Commun. 1977, 720. Auch diese waren instabil.

Die geringe Bedeutung der antibakteriell praktisch unwirksamen oder gering wirksamen Oxapenem-3-carbonsäuren mag man auch daran ermessen, daß ihnen in einem 402 Seiten umfassenden Buch über nichtklassische $\beta$-Laktamantibiotika (Chemistry and Biology of $\beta$-Lactam Antibiotics, Vol. 2, ed. by R.B. Morin and M. Gorman, Academic Press, New York 1982) lediglich 5 Seiten gewidmet sind (Seiten 381-385).

Noch viel geringeres Interesse wurde den Oxapenem-3-carbonsäuren in den folgenden Jahren (1982-1986) entgegengebracht, was durch eine vollständige Literatursuche in Chemical Abstracts bestätigt wurde. Unter dem systematischen Namen 4-Oxa-1-azabicyclo[3.2.0]hept-2-en-2-carbonsäure wurden gefunden, daß die Forschung auf diesem Gebiet ständig abnahm: 1977: 3, 1978: 9, 1979: 2, 1980: 6, 1981: 9, 1982: 2, 1983: 5, 1984: 2, 1985: 0, 1986: O Publikationen. Die Oxapenem-3-carbonsäuren waren also wegen ihrer geringen Stabilität und wegen ihrer geringen antibakteriellen Wirksamkeit für die Fachwelt uninteressant geworden. Dieses geringe Interesse an den Oxapenem-3-carbonsäuren verglichen mit demjenigen an anderen nichtklassischen $\beta$-Lactamantibiotika zeigt, daß ein Vorurteil der Fachwelt gegen die Brauchbarkeit und Wirksamkeit der Stoffklasse der Oxapenem-3-carbonsäuren z.Zt. besteht.

Die Stabilität von $\beta$-Laktamantibiotika ist seit jeher ein zentrales Problem dieser Wirkstoffklasse. So sind beispielsweise im Zweiten Weltkrieg hunderttausende von Soldaten an Wundinfektionen gestorben, weil wegen der Instabilität des Penicillins nicht genügend Material hergestellt werden konnte, um die Erkrankten zu heilen. Erst später mit der Entdeckung der stabilieren, kristallinen Penicilline (Penicillin V und Penicillin

G) gelang die Produktion aus Schimmelpilzen im Tausend-Tonnen-Maßstab.

Auch bei den nichtklassischen B-Laktamantibiotika spielt die Stabilität eine wichtige Rolle: Thienamycin, das z.Zt. wirksamste natürliche Antibiotikum "in vitro" ist sehr hydrolyseempfindlich und deshalb als Therapeutikum nicht anwendbar. Später erst ist ein geeignetes stabileres Derivat (Formiminothienamycin = MK-0787) hergestellt worden (Lit.: Recent Advances in the Chemistry of ß-Lactam Antibiotics, ed. by G.I.Gregory, The Royal Society, London, Seite 249 (1981)).

Die herkömmlichen Oxapenem-3-carbonsäuren sind sehr instabile Stoffe. Es bestand deshalb ein Bedarf auch in dieser Stoffklasse, stabile Derivate herzustellen mit stark verbesserter antibakterieller Wirkung, die sich im wäßrigen Medium lange genug halten, damit sie den Wirkungsort unzuersetzt erreichen, um die pathogenen Bakterien abzutöten.

Oxapenem-3-carbonsäuren der Formeln I und II sind viel stabiler als die früher bekanntgewordenen Verbindungen. Exakte Messungen bei physiologischen Bedingungen, d.h. im wäßrigen Phosphatpuffer bei pH 7,4 und 37° C mit Hilfe der UV-Spektroskopie zeigten eine überraschende Abhängigkeit der Stabilität der Verbindungen III von den Substituenten $R^1$, $R^2$, $R^3$, $R^4$ und $R^5$.

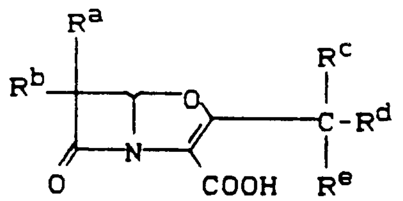

(III)

| | Verbindung (III) | Hydrolyse-Halbwertszeit bei pH 7.4, 37° C (Maß für die Stabilität) |
|---|---|---|
| (a) | $R^a$, $R^b$, $R^c$, $R^d$, $R^e$ = $CH_3$ | 30 Stunden |
| (b) | $R^a$, $R^b$, $R^c$, $R^d$ = $CH_3$; $R^{e\prime}$ = H | 2 Stunden |
| (c) | $R^a$, $R^b$, $R^c$ = $CH_3$; $R^d$, $R^e$ = H | 70 Minuten |
| (d) | $R^a$, $R^b$ = $CH_3$; $R^c$, $R^d$, $R^e$ = H | 50 Minuten |
| (e) | $R^a$, $R^b$, $R^c$, $R^d$, $R^e$ = H | wenige Minuten |

Verbindung IIIa ist identisch mit I ($R^1$, $R^2$, $R^3$, $R^4$, $R^5$ = $CH_3$.

Mit diesen Messungen wird erstmals bewiesen, daß über Kohlenstoff gebundene Gruppen $R^c$, $R^d$, $R^e$ eine bedeutende Stabilisierung der Oxapenem-3-carbonsäuren bewirken. Schon eine einzige Gruppe $R^c$, $R^d$ oder $R^e$ = H führt zu einer drastischen Erniedrigung der Stabilität.

Die in früheren Patentanmeldungen (z.B. in EP 18305) als bevorzugt bezeichnete Verbindung IIIe hydrolysiert in wenigen Minuten und könnte durch die Blutbahn (pH 7,4, 37° C) niemals effizient an den Wirkort unbeschadet transportiert werden. Aber auch in vitro ist IIIe wegen sofortiger Hydrolyse antibakteriell praktisch unwirksam. Bei Verwendung von Staphylococcus aureus DSM 1104 wurden nach Auftragen von 250 µg IIIe nur ein Hemmhof von wenigen mm festgestellt im Agar-Diffusionstest.

Die Verbindungen der Formeln I und II besitzen eine hohe Wirksamkeit gegen Staphylococcus aureus. Gewisse Vertreter sind ebenso wirksam gegen grampositive wie gegen gramnegative Keime und resistente Bakterien. So ergibt die Verbindung (I) ($R^1$, $R^2$ = H; $R^3$, $R^4$, $R^5$ = $CH_3$), die sich vom antibakteriell praktisch unwirksamen IIIe nur durch den Mehrbesitz von drei Methylgruppen auszeichnet, folgende Hemmhof-Durchmesser nach Auftragen von 200 µg Substanz im Agar-Diffusionstest:

| | |
|---|---|
| Staph. aureus DSM 1104 | 45 mm |
| Staph. aureus 012484/77 (Penicillin- und Cephalosporin-resistent) | 47 mm |
| Escherichia coli DSM 1103 | 41 mm |

Durch geeignete Substitution konnte die Wirksamkeit gegenüber gewissen Keimen erheblich gesteigert werden. So zeigt beispielsweis die Verbindung (I) ($R^1$ = H; $R^2$ = $CH_2OH$; $R^3$, $R^4$, $R^5$ = $CH_3$) Hemmhof-

Durchmesser nach Auftragen von nur 10 µg Substanz:

| | |
|---|---|
| Staph. aureus DSM 1104 | 30 mm |
| Staph. aureus 012484/77 | 32 mm |
| Escherichia coli DSM 1103 | 30 mm |
| Escherichia coli W 3110 R6K (TEM 1) (ß-Lactamase bildend) | 29 mm |

Die obigen Daten zeigen, daß die bisher als antibakteriell praktisch unwirksam und folglich als uninteressant betrachtete Klasse der Oxapenem-3-carbonsäuren erstmals unter die der wirksamsten antibakteriellen Mittel überhaupt aufrückt. Penicillin V (130 µg) zeigte nur eine starke Hemmwirkung gegen Staph. aureus 1104 (42 mm) und eine minimale Wirkung gegen E. coli DSM 1103 (13 mm). Die beiden anderen Keime wurden nicht gehemmt. Vergleichbare Daten über die antibakterielle Wirksamkeit des natürlichen Antibiotikums Thienamycin finden sich in Journ. Amer. Chem. Soc. 100, 8004 (1978): Die Hemmhof-Durchmesser nach Auftragen von 25 µg Substanz betrugen hier bei Verwendung von ähnlichen Keimen 28-41 mm.

Diese Antibiotika sind gegenüber vielen grampositiven, gramnegativen, Penicillin-resistenten und Cephalosporin-resistenten Bakterien wirksam. Die Voraussetzung für diese hohe Wirksamkeit und Anwendbarkeit wird geschaffen durch die Trisubstituion des exocyclischen, allylischen Kohlenstoffatoms von I bzw. II mit drei via Kohlenstoffatome gebundenen Gruppen $R^3$, $R^4$ und $R^5$. Die überlegende antibaketrielle Wirksamkeit dieser Oxapenem-3-carbonsäuren konnte nach dem Stand der Wissenschaft nicht in diesem Maße erwartet werden.

Die Verbindungen der obigen Formeln I und II werden zweckdienlich nach dem folgenden Schema hergestellt:

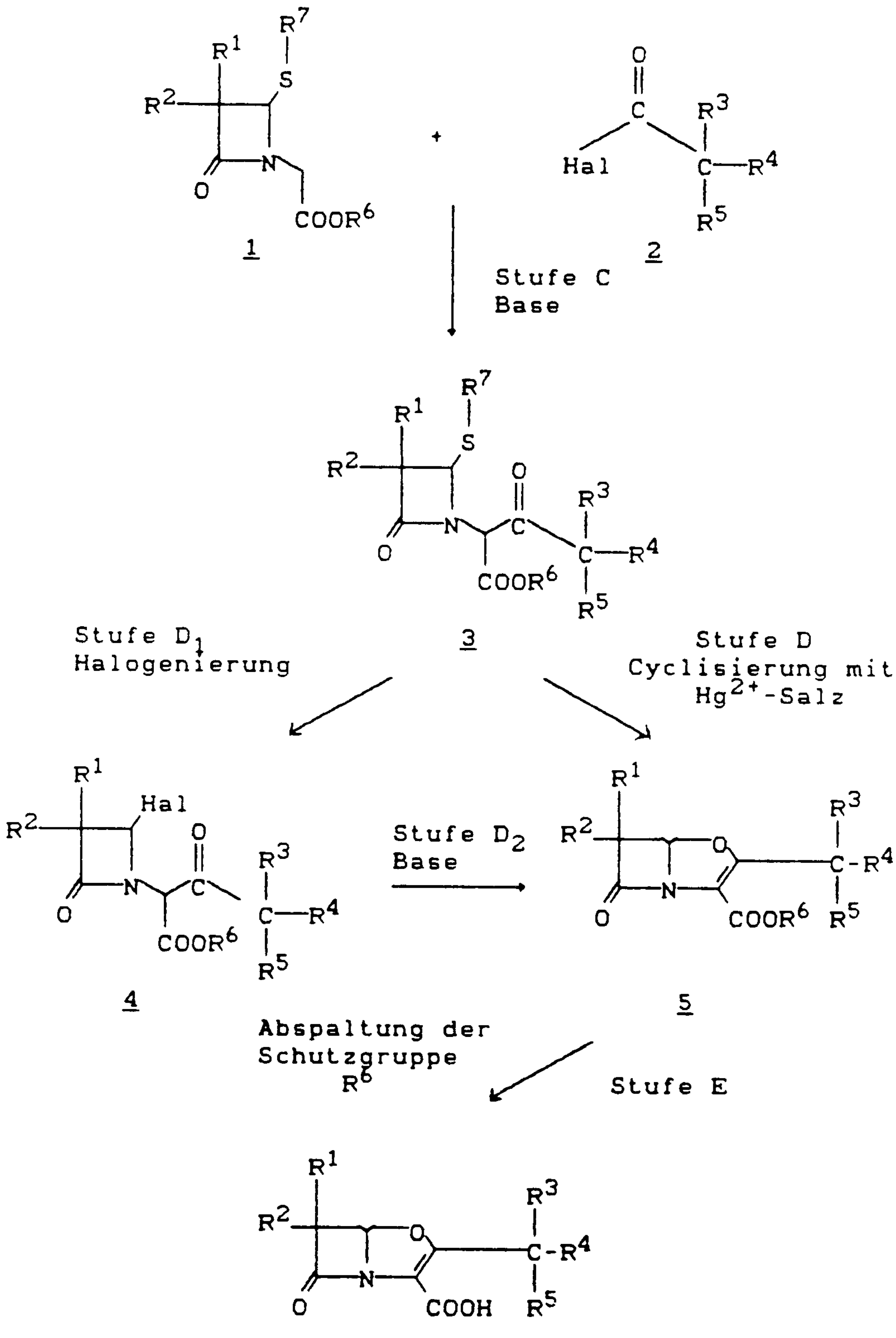
Stufe C
Base
Stufe D1
Halogenierung
Stufe D
Cyclisierung mit
Hg2+-Salz
Stufe D2
Base
Abspaltung der
Schutzgruppe
R6
Stufe E

1 2 3 4 5

6 (I)

Verbindungen II werden zweckdienlich nach dem folgenden Reaktionsschema gewonnen:

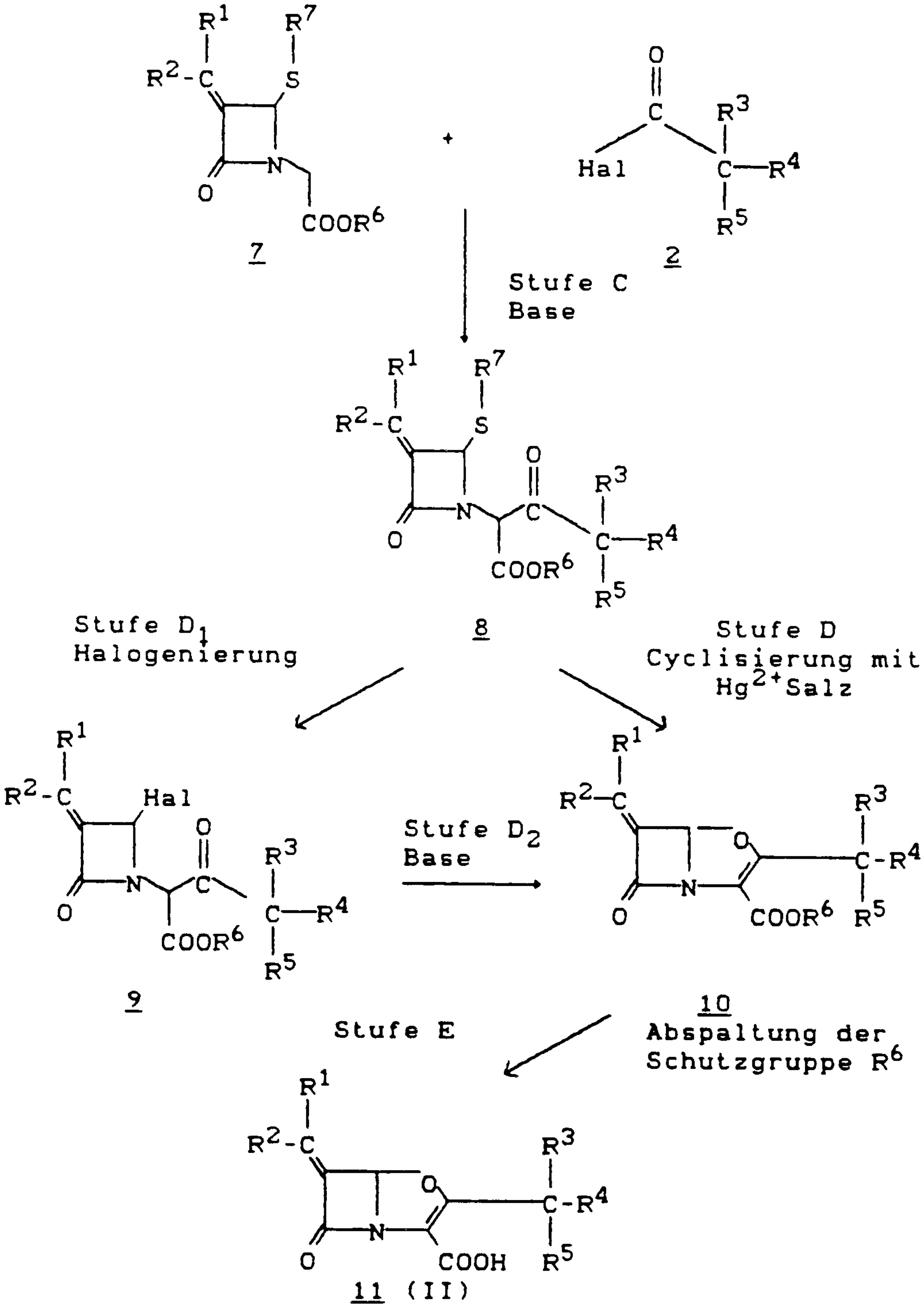


7, 2, 8, 9, 10, 11 (II)

worin in beiden Reaktionsschemata $R^1$, $R^2$, $R^3$, $R^4$ und $R^5$ die oben angegebenen Definitionen besitzen, $R^6$ eine leicht entfernbare Schutz- bzw. Maskierungsgruppe bedeutet und wobei $R^6$ ebenfalls der Molekülteil eines pharmazeutisch annehmbaren Esters sein kann. Typischerweise ist die Schutzgruppe $R^6$ eine Acylgruppe, wie niedrig-Alkanoyl, Aralkylcarbonyl o.ä, wie Acetyl, Brom-tert.-butoxycarbonyl, Benzyloxycarbonyl, Formyl, Trifluoracetyl u.ä., oder eine Trialkylsilylgruppe, wie Trimethylsilyl oder tert.-Butyldimethylsilyl; und typischerweise ist die Schutzgruppe $R^6$ eine substituierte oder unsubstituierte Alkyl-, Aralkyl-, Alkenyl- oder ähnliche Gruppe, wie Benzyl, o-Nitrobenzyl, p-Nitrobenzyl, Trimethoxybenzyl, 2-Oxopropyl, 2-Oxo-2-phenylethyl, Allyl, 2-Cycloethyl, 2-Trimethylsilyloxyethyl, 2,2,2-Trichlorethyl, Pivaloyloxymethyl, Brom-tert.-butyl u.ä.

Typischerweise ist $R^7$ eine substituierte oder unsubstituierte, verzweigte oder unverzweigte Alkylgruppe, Aralkylgruppe, Arylgruppe, Heteroaryl oder Heteroaralkylgruppe, wobei die Substituenten Niederalkyl, Acyloxy, Chlor, Brom, Nitro, Niederalkyloxy, Cyano u.ä. bedeuten und die Heteroatome des Heteroaryl- oder Heteroaralkylteils ausgewählt werden aus der Gruppe Sauerstoff, Stickstoff und Schwefel. Besonders typische Reste $R^7$ sind Methyl, Ethyl, Propyl, Isopropyl, Butyl, Phenyl, Tolyl, Benzyl, Triphenylmethyl, tert.-Butyl, 2-Mercaptobenzthiazolyl u.ä.

Obige Reaktionsdiagramme werden im folgenden näher in Worten erläutert. Ein geeignet substituiertes Azetidinon (1) oder (7) wird mit dem Säurehalogenid (2) mit etwa 1 bis 2 Äquivalenten einer Base wie Butyllithium, Lithiumdiisopropylamid oder Lithium-bis-(trimethylsilylamid) u.ä., bei niedriger Temperatur von etwa -70° C bis 0° C innerhalb etwa einer Stunde zu 3 bzw. 8 umgesetzt. Die Identität des Lösungsmittels ist nicht kritisch, vorausgesetzt nur, daß die Reaktionsteilnehmer löslich sind und es bei der Umsetzung inert oder im wesentlichen inert ist. Bei der Reaktion (1 → 3) bzw. (7 → 8) wird zweckdienlich Tetrahydrofuran, Dioxan, Glyme, Dimethylformamid oder ein Gemisch dieser Lösungsmittel mit Hexan verwendet.

Die Reaktion (3 → 4) bzw. (8 → 9) kann nach irgend einem bekannten Halogenierungsverfahren durchgeführt werden. Geeignete Halogenierungsmittel sind Chlor, Brom, Iod, Sulfurylchlorid u.ä. Bei einem bevorzugten Halogenierungsverfahren wird 3 bzw. 8 in einem inerten Lösungsmittel wie beispielsweise Tetrachlorkohlenstoff, Toluol oder Methylenchlorid mit 1 bis 2 Äquivalenten Chlor behandelt. Typischerweise weird diese Reaktion bei einer Temperatur von etwa -70° C bis 0° C während 0,5 bis 2 Stunden durchgeführt.

Bei der Reaktion (4 → 5) bzw. (9 → 10) wird 4 bzw. 9 mit etwa 1 bis 2 Äquivalenten einer Base wie beispielsweise Natriummethoxid, Kalium-tert.-butoxid, Natriumphenolat, Natriumthiophenolat, Diazabicycloundecen u.ä. in einem geeigneten inerten Lösungsmittel wie beispielsweise Toluol, Tetrahydrofuran oder Dimethylformamid zu 5 bzw. 10 umgesetzt. Die typische Reaktionszeit beträgt etwa 30 Minuten bis 2 Stunden, die typische Reaktionstemperatur etwa -70° C bis Raumtemperatur.

Bei der direkten Cyclisierungsreaktion (3 → 5) bzw. (8 → 10) wird 3 bzw. 8 mit 1-3 Äquivalenten eines Quecksilber(II)salzes wie beispielsweise Quecksilberchlorid in einem geeigneten inerten Lösungsmittel wie beispielsweise Glyme, Dioxan oder Tetrahydrofuran zu 5 bzw. 10 umgesetzt. Auch Gemische zweier oder mehrerer Quecksilber(II)salze, beispielsweise das 1:1-Gemisch von Quecksilber(II)oxid und Quecksilber(II)-chlorid werden typischerweise benutzt. Die typische Reaktionstemperatur beträgt 60-100° C, die typische Reaktionszeit 2 bis 20 Stunden.

Die Entfernung der Schutzgruppe (5 → 6) bzw. (10 → 11) erfolgt nach an sich wohlbekanntem Verfahren, wie katalytische Hydrierung, Hydrolyse, Reduktion, nucleophile Substitution, Solvolyse u.ä. Geeignete Hydrierungskatalysatoren für die Schutzgruppenabspaltung umfassen die Platinmetalle und ihre Oxide, Raney-Nickel, Palladium auf Kohle, u.ä., geeignete Lösungsmittel für die Hydrierung sind Methanol, Ethanol, Essigsäureethylester/$H_2O$, Ethanol/$H_2O$ u.ä. in Abwesenheit von Wasserstoff bei einem Druck von 1 bis 50 at. Die Hydrierung dauert typischerweise 5 Minuten bis 2 Stunden bei einer Temperatur von 0-25° C und wird gegebenenfalls in Gegenwart einer schwachen Base, beispielsweise Natriumhydrogencarbonat durchgeführt. Beim hydrolytischen Abbau der Schutzgruppe wird 5 bzw. 10 in einem geeigneten Lösungsmittel wie beispielsweise Tetrahydrofuran oder Tetrahydrofuran/$H_2O$ mit 1 Äquivalent Base wie z.B. verdünnter wäßriger Natronlauge u.ä. versetzt. Typischerweise dauert die Reaktion 5-60 Minuten; die Reaktionstemperatur beträgt -30 bis 0° C. Beim reduktiven Abbau der Schutzgruppe wird 5 bzw. 10 in einem geeigneten Lösungsmittel, beispielsweise Essigsäure/Wasser mit 1 - 3 Äquivalenten eines Reduktionsmittels, beispielsweise Zinkstaub u.ä. versetzt. Typischerweise dauert die Reaktion 30 Minuten bis 2 Stunden; die Reaktionstempertur beträgt -30° C bis Raumtemperatur. Beim Abbau der Schutzgruppe durch nucleophile Substitution wird 5 bzw. 10 in einem inerten Lösungsmittel, beispielsweise Tetrahydrofuran mit einem nucleophilen Agens, beispielsweise Tetrabutylammoniumfluorid u.ä. umgesetzt. Typischerweise dauert die Reaktion 30 Minuten bis 2 Stunden; die Reaktionstemperatur beträgt -30° C bis Raumptemperatur. Beim Abbau der Schutzgruppe durch Solvolyse wird 5 bzw. 10 in einem geeigneten Lösungsmittel, beispielsweise Tetrahydrofuran mit 1 bis 2 Äquivalenten einer Lewissäure, beispielsweise Aluminiumtrichlorid versetzt und anschließend mit einem solvolysierenden Lösungsmittel, beispielsweise Wasser versetzt. Typischerweise dauert die Reaktion 30 Min. bis 2 Stunden; Reaktionstemperatur beträgt 0° C bis Raumtemperatur.

Die dreifach substituierten Acetylchloride (2) sind teilweise im Handel erhältlich wie z.B. Pivaloylchlorid oder 3-Chlorpivaloylchlorid oder sie sind literaturbekannt wie z.B. 2-Methyl-2-phenylpropanoylchlorid (Helv. Chim. Acta 54, 870 (1971); J. Org. Chem. 39, 3268 (1974)) oder 3-Acetoxypivaloylchlorid (Bull. Chem. Soc. France 31, 125 (1904); J. Org. Chem. 24, 1228 (1959)) oder sie können in Analogie zu ähnlichen bekannten Stoffen hergestellt werden wie z.B. 2-Methyl-2-thienylpropanoylchlorid nach der Synthesevorschrift für das Phenylderivat.

Die Verbindungen der Formeln 3 bzw. 8 und 4 bzw. 9 liegen wegen der Trisubstitution des α-Kohlenstoffatoms durch die via Kohlenstoffatome gebundenen Gruppen $R^3$, $R^4$ und $R^5$ ausschließlich als Ketone vor, was sich durch das Fehlen von NMR-Enol-Resonanzen bei 11.6 ppm (Tetrahedron 38, (16), 2489-2504 (1982), Seite 2490) und die Anwesenheit einer Keton-Carbonylbande bei ~ 1720 $cm^{-1}$ und einer Bande eines gesättigten Carbonsäureesters bei ~ 1755 $cm^{-1}$ im IR-Spektrum aufgenommen in Methylenchlorid äußerte. Die Ketonstruktur ist auch durch mangelnde Reaktivität bewiesen: So ergeben diese Verbindungen , in Methylenchlorid auf ein Filterpapier aufgetragen und mit wäßriger Eisen(III)chlorid-Lösung besprüht, keine violette Färbung. Die Ketone der Formeln 3 bzw. 8 und 4 bzw. 9 werden auch nicht, mit Diazomethanlösung in Ether versetzt, in die Enolether umgewandelt. Alle diese Befunde stehen im Gegensatz zu früher bekanntgewordenen Zwischenprodukten ohne die Trisubstitution; diese lagen ausschließlich oder hauptsächlich als Enole vor (z.B. in EP 0 018 305 Al, Seite 3; Tetrahedron 38, (16), 2490 (1982); J.C.S. Chem. Comm. 1977, 720, J.C.S. Chem. Comm. 1977, 905).

Zwar wurden in früheren Patentanmeldungen (z.B. in EP 0 018 305 Al) Oxapenem-3-carbonsäuren mit verzweigten aliphatischen Resten in 2-Stellung erwähnt. Weil diese aber aus Enolen hergestellt wurden, kann es sich nicht um die Verbindungen I oder II handeln.

Bei Verwendung von chiralen Azetidin-2-onen der Formeln 1 bzw. 7 mit der 4R-Konfiguration werden gegebenenfalls nach den beschriebenen Reaktionsschemata (1 → 6) bzw. (7 → 11) chirale 1-Oxapen-2-em-3-carbonsäuren (I) bzw. (II) erhalten, die ebenfalls die 4R-Konfiguration besitzen.

Eine Variante der Synthese von Verbindungen (I) und (II) ergibt sich gegebenenfalls durch eine Unwandlung der Gruppen $R^3$, $R^4$, $R^5$ auf der Stufe des Ketons 3 bzw. 8. So kann beispielsweise eine Gruppe $R^3$ = Alkyl-Cl mit Nucleophilen wie z.B. Tetraalkylammoniumazid in eine Gruppe $R^3$ Alkyl-$N_3$ übergeführt werden. Typisches Lösungsmittel für die beispielhafte Reaktion ist DMF. Typische Reaktionstemperatur ist 0° C bis 80° C und die Reaktion dauert typischerweise 2-48 Stunden.

Eine vorteilhafte Variante der Synthese von Verbindungen (I) und (II) ergibt sich gegebenenfalls durch eine mehrfache Abspaltung von ausgewählten Schutzgruppen in der Stufe (5 → 6) bzw. (10 → 11). So können beispielsweise geschützte Hydroxyalkylgruppen $R^1$, $R^2$ aber auch geschützte Hydroxyalkyl- oder geschützte Aminoalkylgruppen $R^3$, $R^4$, $R^5$ bei der Entfernung der Schutzgruppe $R^6$ gleich mit freigesetzt werden.

Im folgenden wird die Synthese des Ausgangsmaterials 1 beschrieben. 1 wird nach an sich bekannten Verfahren aus 4-Acyloxyazetidin-2-onen der Formel 12 oder aus Sulfonylazetidin-2-onen der Formel 13 auf folgendem Weg hergestellt:

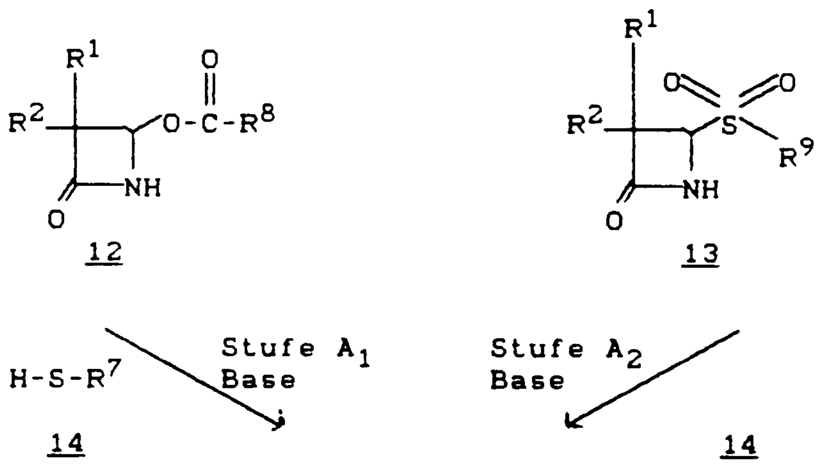


12

13

$H-S-R^7$

14

14

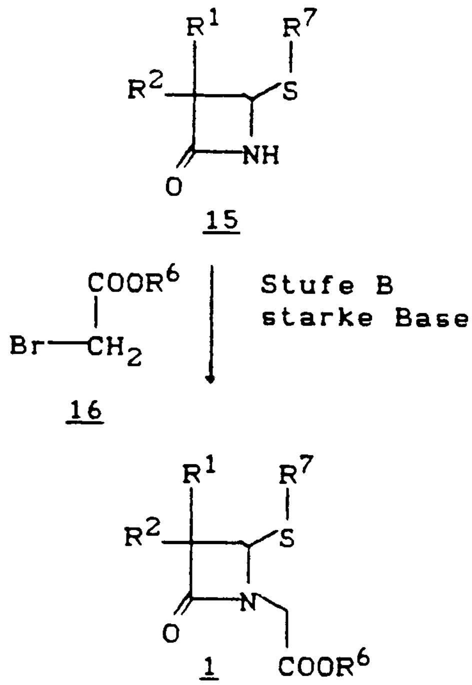


15

16

1

worin $R^1$, $R^2$, $R^7$ und $R^6$ die obengenannten Bedeutungen haben und $R^8$ eine Alkyl- oder Arylgruppe wie beispielsweise Methyl oder Phenyl bedeutet, $R^9$ ist typischerweise eine Alkyl- oder Arylgruppe, wie beispielsweise Methyl oder Phenyl oder eine Hydroxyalkylgruppe wie beispielsweise 2-Hydroxyethyl, 2-Hydroxyisopropyl, 2-Hydroxy-1-phenylethyl oder 2-Hydroxy-tert.-butyl u.ä..

Bei der Reaktion (12 → 15) bzw., (13 → 15) wird 12 bzw. 13 mit 1-1.5 Äquivalenten eines Mercaptens (14) in einem geeigneten Lösungsmittel wie Tetrahydrofuran, Tetrahydrofuran/$H_2O$ oder Isopropanol/$H_2O$ mit einer Base wie Diazabicycloundecen oder Natronlauge u.ä. zu 15 umgesetzt. Typischerweise beträgt die Reaktionstemperatur -30° C bis Raumtemperatur und die Reaktionsdauer etwa 30 Minuten bis 4 Stunden.

Bei der Reaktion (15 → 1) wird 15 mit einem geeigneten Bromessigsäurester (16) in einem inerten Lösungsmittel wie Tetrahydrofuran oder Hexan mit einer starken Base wie Butyllithium, Kalium-tert.-butoxid, Lithiumdiisopropylamid oder Lithium-bis-(trimethylsilylamid) u.ä. zu 1 umgestzt. Typische Reaktionstemperaturen sind etwa -70-0° C, typische Reaktionszeiten 30 Minuten bis 2 Stunden.

Die Verbindungen 12 sind aus Chlorsulfonylisocyanat und Vinylestern nach Ann. Chem. 1974, 539 herstellbar, aber auch Synthesen, die von Penicillin ausgegeben, sind bekannt (z.B. in Recent Advances in the Chemistry of ß-Lactam Antibiotics, ed. by G.I. Gregory, The Royal Society of Chemistry, London, Seiten 330-348 (1981)). Verbindungen 13 sind entweder aus 12 herstellbar nach Ann. Chem. 1974, 539 oder nach Journ. Amer. Chem. Soc., 102, 2039 (1980) bzw. Recent Adv. in the Chem. of ß-Lactam Antibiotics, ed. by G.I. Gregory, the Royal Society of Chemistry, London, Seite 368-378 (1981)), aber auch Verfahren der Herstellung von 13 aus Penicillin sind bekannt (z.B. Tetrahedron Lett. 22, 4141-4144 (1981)).

Bei Verwendung von chiralen Azetidinonen 12 bzw. 13 mit der 4R-Konfiguration entstehen Verbindungen 1 mit der gleichen 4R-Konfiguration.

Im folgenden wird die Synthese des ungesättigten Ausgangsmaterials 7 beschrieben. 7 wird zweckmäßigerweise nach dem folgenden Reaktionsschema hergestellt:

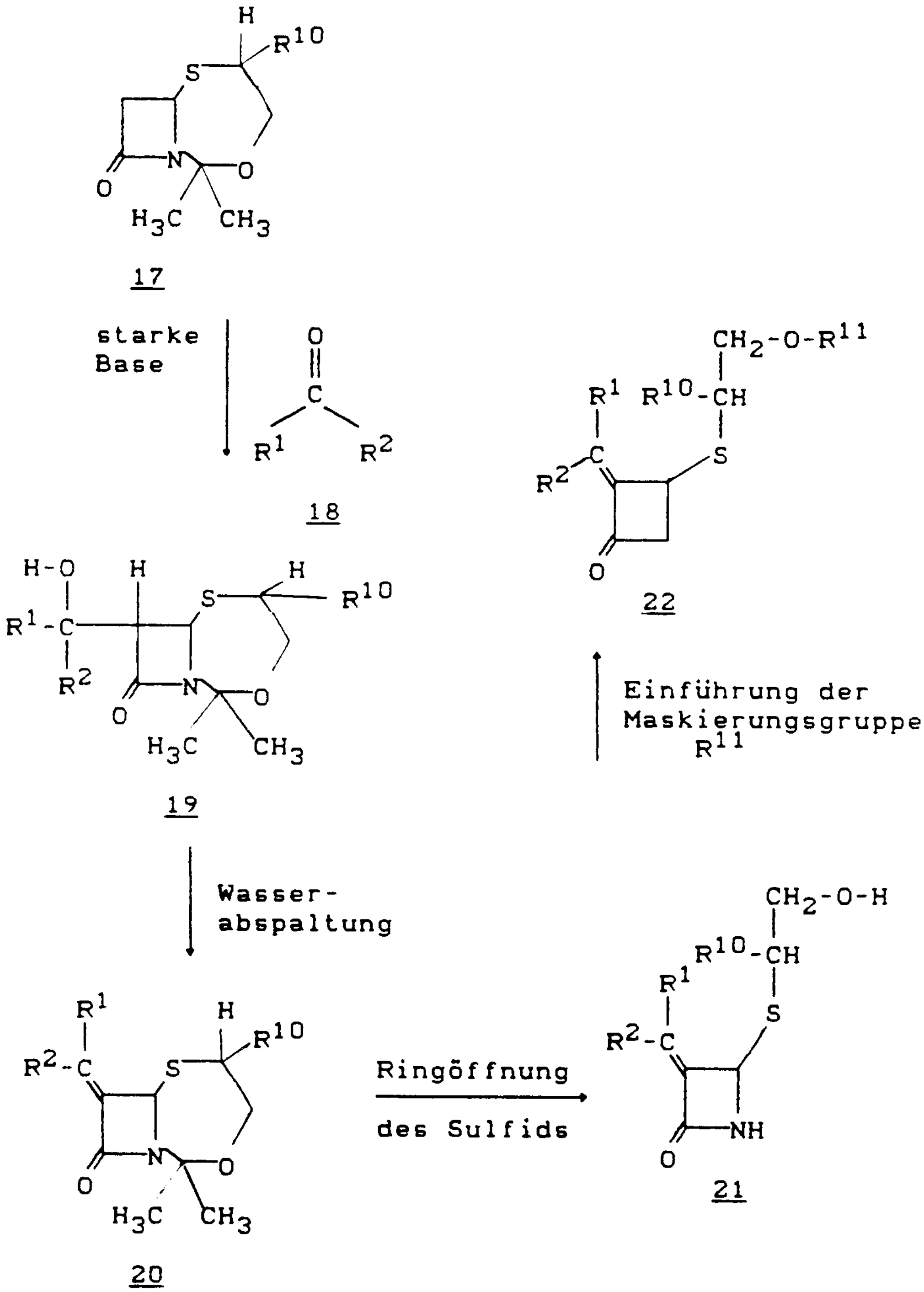


17

18

19

20

21

22

20

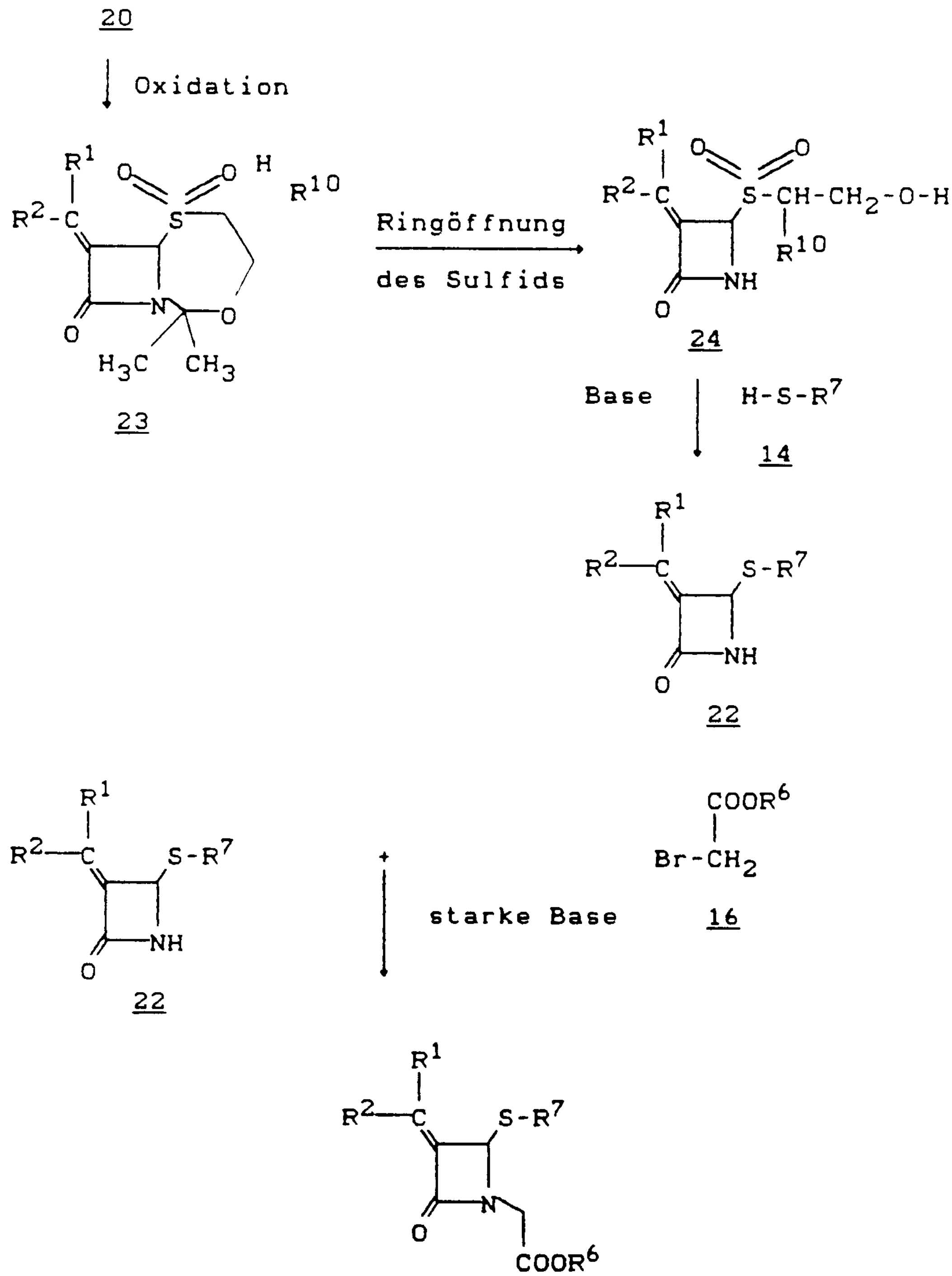


23

24

14

22

22

16

7

worin $R^1$, $R^2$, $R^6$ und $R^7$ die oben angegebenen Definitionen besitzen, $R^{10}$ Wasserstoff, eine Alkyl- oder Arylgruppe bedeutet wie beispielsweise Methyl oder Ethyl und $R^{11}$ eine leicht einführbare Maskierungsgruppe wie Alkyl, Aryl, Aralkyl, Acyl, Trialkylsilyl. Typischerweise ist $R^{11}$ Benzyl, Acetyl, Benzoyl, Trimethylsilyl- oder tert.-Butyldimethylsilyl u.ä. Die Identität der Maskierungsgruppe ist jedoch nicht besonders kritisch, da sie nicht als solche abgespalten werden muß aber trotzdem auf einer spätereren Reaktionsstufe, bei der Halogenierung (8 → 9) oder der Cyclisierung mit Quecksilber(II)-salz (8 → 10), als Teil eines abgespalteten Moleküls wieder verwendet.

Bei der Reaktion (17 → 19) wird 17 in einem inerten Lösungsmitel, wie beispielsweise Tetrahydofuran oder Tetraydrofuran/Hexan u.ä. mit 1 bis 1.2 Äquivalenten einer starken Base, wie beispielsweise Lithiumdiisopropylamid u.ä. versetzt und anschließend mit dem Keton 18 zu 19 umgesetzt. Typischerweise beträgt die Reaktionstemperatur -70° C bis 0° C; die Reaktionszeit 30 Minuten bis 2 Stunden.

Bei der Wasserabspaltungs-Stufe (19 → 20) wird 19 in einem inerten Lösungsmittel wie beispielsweise Tetrahydrofuran mit 1 bis 1.5 Äquivalenten eines Säurechlorids wie beispielsweise Thionylchlorid, p-Toluolsulfonsäurechlorid, Methansulfonsäurechlorid oder Acetylchlorid o.ä. und 1 bis 5 Äquivalenten einer Base wie Pyridin, Triethylamin, N,N-Dimethylaminopyridin o.ä. versetzt und der intermediäre Ester anschließend in einem inerten Lösungsmittel, wie beispielsweise Tetrahydrofuran mit einer starken Base wie Kalium.-tert.-butoxid oder Diazabicycloundecen o.ä. zu 20 umgesetzt. Typischerweise betragen die Reaktionstemperaturen bei beiden Reaktionsschritten, d.h. bei der Veresterung und bei der Elimimation -30° C bis +50° C. Die Reaktionsdauer der Veresterung beträgt je nach Basenstärke der verwendeten Base 2 Stunden bis 48 Stunden. Die Reaktionsdauer der Elimination beträgt etwa 30 Minuten bis 2 Stunden. Einfacher gelingt die Wasserabspaltungs-Stufe durch Erhitzen von 19 in einem inerten Lösungsmittel wie Toluol u.ä. im Wasserabscheider mit Hilfe eines Katalysators, wie beispielsweise p-Toluolsulfonsäure oder p-Toluolsulfochlorid. Bei der Rückflußtemperatur des Toluols beträgt die Reaktionszeit typischerweise 2-10 Stunden.

Bei der Ringöffnungs-Stufe des Sulfids (20 → 21) wird 20 in einem sauren Lösungsmittel, wie beispielsweise Essigsäure/$H_2O$ o.ä. erhitzt. Bei der Rückflußtemperatur von ca. 110° C dauert die Reaktion typischerweise 30 Minuten bis 2 Stunden.

Die Einführung der Maskierungsgruppe $R^{11}$ (21 → 22) erfolgt durch Umsatz von 21 mit 1 bis 1.3 Äquivalenten eines geeigneten leicht einführbaren Alkylierung- oder Acylierungsmittel wie beispielsweise Benzylchlorid, Benzylbromid, Acetylchlorid, Benzoylchlorid, Trimethylchlorsilan oder tert.-Butyldimethylchlorsilan in Gegenwart einer Base, wie Kalium-tert.-butoxid, Triethylamin, N,N-Dimethylaminopyridin, Pyridin, Imidazol o.ä. in einem inerten Lösungsmittel wie Tetrahydrofuran oder Dimethylformamid u.ä. Typischerweise beträgt die Reaktionstemperatur etwa -30° C bis Raumtemperatur.

Die Oxidations-Stufe (20 → 23) erfolgt durch Umsatz von 20 mit einem an sich bekannten Oxidationsmittel, das zur Sulfoxidierung verwendet werden kann, wie beispielsweise Kaliumpermanganat, Wasserstoffperoxid, m-Chlorpherbenzoesäure o.ä. Typischerweise wird eine Lösung von 20 in einem inerten Lösungsmittel, wie beispielsweise Methylenchlorid, Chloroform oder Aceton mit 2 bis 2,5 Äquivalenten eines Oxidationsmittels wie m-Chlorperbenzoesäure zu 23 umgesetzt. Typischerweise beträgt die Reaktionstemperatur -30° C bis Raumtemperatur und die Reaktionszeit 30 Minuten bis 2 Stunden.

Bei der Ringöffnungs-Stufe des Sulfons (23 → 24) wird 23 in einem sauren Lösungsmittel, wie beispielsweise Essigsäure/$H_2O$ o.ä. erhitzt. Bei der Reaktionstemperatur von ca. 110° C dauert die Reaktion typischerweise 30 Minuten bis 2 Stunden.

Verbindungen der Formel 24 werden dann mit einem Mercaptan 14 in Gegenwart einer Base, wie beispielsweise Natronlauge und Diazabicycloundecen o.ä. zu 22 umgesetzt. Die Reaktionsbedingungen entsprechen denjenigen der Reaktionsstufe (12 → 15).

Verbindungen der Formel 22 werden mit einem Bromessigsäureester der Formel 16 mit Hilfe einer starken Base, wie beispielsweise Butyllithium, Lithiumdiisopropylamid oder Lithium-bis-trimethylsilyl-amid zu Verbindung 7 umgesetzt. Die Reaktionsbedingungen entsprechen denjenigen der Reaktionsstufe (15 → 1).

Die Verbindungen 17 sind nach Recent Adv. in the Chem. of ß-Lactam Antibiotics, ed. by G.I. Gregory, the Royal Society of Chemistry, London, Seite 368-378 (1981) oder nach Tet. Lett. 22, 4141-4144 (1981) zugänglich. Bei Verwendung von chiralem Ausgangsmaterial 17 mit der 7R-Konfiguration entsteht bei der Umwandlung (17 → 7) chirales 7 mit der gleichen 4R-Konfiguration.

In der allgemeinen Beschreibung der vorliegenden Erfindung sind die Gruppen $R^1$ und $R^2$ bevorzugt ausgewählt aus: Wasserstoff, Alkyl, geschütztem oder ungeschütztem Hydroxyalkyl oder geschütztem oder ungeschütztem Dihydroxyalkyl mit bis zu 6 Kohlenstoffatomen, $R^3$, $R^4$ und $R^5$ werden bevorzugt ausgewählt aus substituiertem oder unsubstituiertem Alkyl, Alkenyl, Alkinyl, Cycloalkyl, Alkylcycloalkyl, Cycloalkylalkyl, Alkenylcycloalkyl, Cycloalkenylalkyl, Aryl, Aralkyl, Aralkenyl, Aralkinyl, worin die vorhergehenden Alkyl-, Alkenyl-, oder Alkinylteile 1 bis 6 Kohlenstoffatome, die Cycloalkyl-oder Cycloalkenyl-Molekülteile 3 bis 6 und die Aryl-Molekülteile 6 bis 10 Kohlenstoffatome enthalten, Heteroaryl, Heteroaralkyl, Heteroaralkenyl, Heteroaralkinyl, Alkylheteroaryl, Heterocyclyl, Heterocyclylalkyl, Heterocyclylalkenyl, Heterocyclylalkinyl, Alkylheterocyclyl, worin die vorhergehenden Alkyl-, Alkenyl-, oder Alkinyl-Molekülteile 1 bis 6 Kohlenstoffatome enthalten und der heteroaromatische oder heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und wobei die Substituenten der oben aufgeführten Gruppen sein können: Geschütztes oder ungeschütztes Hydroxy, Hydroxyalkoxy, Aminoalkyloxy, Amidinoalkyloxy, Alkyloxy, Acyloxy, Aryloxy, Heteroaryloxy, Heterocyclyloxy, Carbamoyl, Carbamoyloxy, Thiocarbamoyl, Thiocarbamoyloxy, Alkylcarbamoyloxy, Alkylthiocarbamoyloxy, Mercapto, Alkylthio, Hydroxyalkylthio, Aminoalkylthio, Amidinoalkylthio, Acylthio, Arylthio, Alkylheteroarylthio, Hydroxyalkylheteroarylthio, Heterocyclylthio, Alkylthiocarbamoylthio, geschütztes oder ungeschütztes Amino oder Monoalkylamino, Dialkylamino, Oxo, geschütztes oder ungeschütztes Oximino oder Alkylimino, Tetraalkylammonium, Cycloalkylamino, Arylamino, Heteroarylamino, Heterocyclamino, Acylamino, Amidino, Alkylamidino, Guanidino, Alkylguanidino, Carbamoylamino, Alkylcarbamoylamino, Thiocarbamoylamino, Alkylthiocarbamoylamino, Nitro, Chlor, Brom, Fluor, Iod, Azido, Cyano, Alkylsulfinyl, Alkylsulfonyl, Sulfonamido, Sulfamoyloxy, Alkylsulfonyloxy oder geschütztes oder ungeschütztes Sulfo, Sulfoxy oder Carboxy, wobei die Substituenten ein- oder mehrfach auftreten und ihr Alkyl-Molekülteil 1 bis 6 Kohlenstoffatome, ihr Aryl-Molekülteil 6 bis 10 Kohlenstoffatome enthält, und wobei der heteroaromatische oder heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff.

Eine besonders bevorzugte Verbindungsklasse ist die, worin $R^1$ und $R^2$ unabhängig voneinander Wasserstoff, Alkyl, geschütztes oder ungeschütztes Hydroxyalkyl oder geschütztes oder ungeschütztes Dihydroxyalkyl bis zu 6 Kohlenstoffatomen bedeuten, $R^3$ und $R^4$ Methyl bedeuten und $R^5$ ausgewählt wird unter den Gruppen:

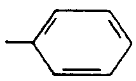
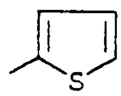
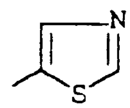

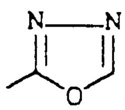
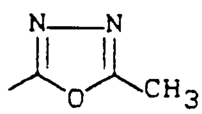
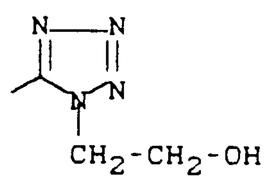

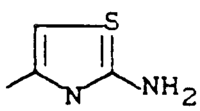
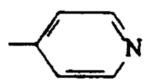
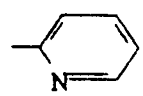

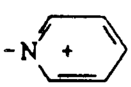
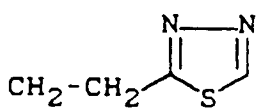
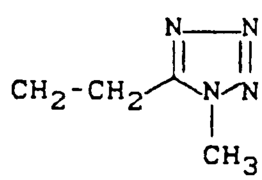

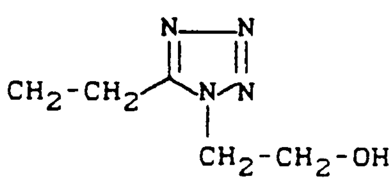
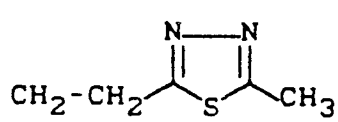

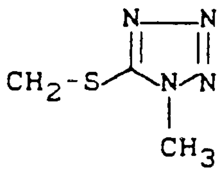

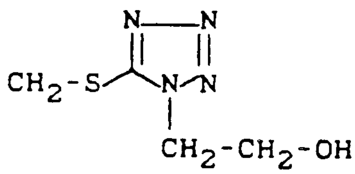

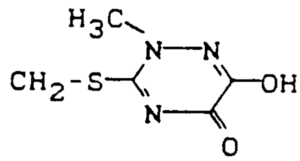

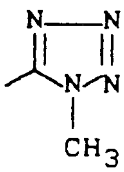

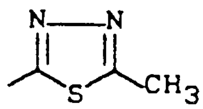

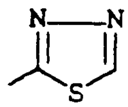

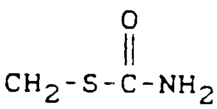

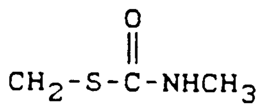

$CH_2-S-CH_3$

$CH_2-S-CH_2-CH_2-NH_2$

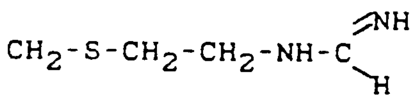

$CH_2-NH_2$

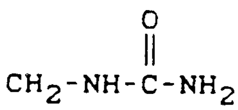

$CH_2-NH-CHO$

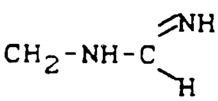

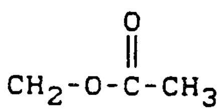

$CH_2-O-CHO$

$CH_2-Cl$

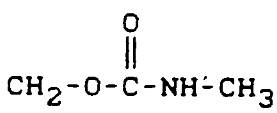

$CH_2-N_3$

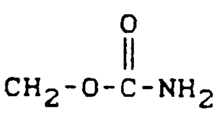

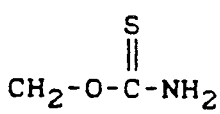

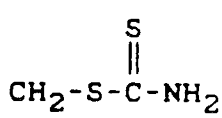

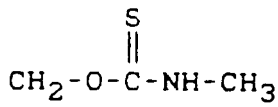
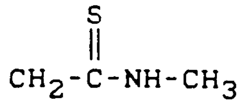
$CH_2-CH_2-NH_2$

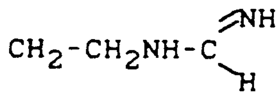
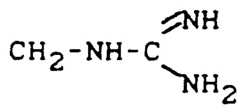
$CH_2-CH_2-CH_2-NH_2$

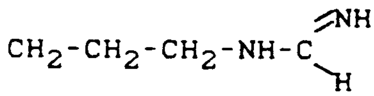
$CH_2-CH_2-COOH$
$CH_2-COOH$

$COOH$
$CH_2-CH_2-CH_2-COOH$
$CH_2-OH$

$CH_2-CH_2-CH_2-OH$
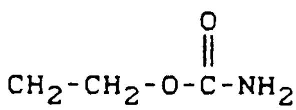
$C\equiv N$

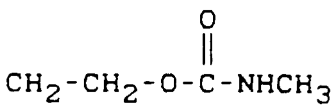
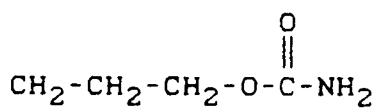

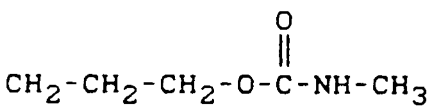
$CH_2-CH_2-CH_2-NH-CHO$ .

Bevorzugte Ester, die als Schutzgruppen verwendet werden, sind solche, worin $R^6$ Benzyl, p-Nitrobenzyl, Methyl, tert.-Butyl, Diphenylmethyl, Trimethylsilyl, tert.-Butyldimethylsilyl, Trichlorethyl bedeutet, oder $R^6$ bedeutet die pharmazeutisch annehmbaren Estermolekülteile, wie Pivaloyloxymethyl, Allyl, Methallyl, 2-Oxoethyl, 2-Oxopropyl, (2-Methylthio)-ethyl oder 3-Buten-1-yl.

Bevorzugte Schutzgruppen der geschützten Hydroxyalkyl und Dihydroxyalkylgruppen $R^1$ und $R^2$ sind Benzyl, p-Nitrobenzyl, Benzyloxycarbonyl, p-Nitrobenzyloxycarbonyl, Trimethylsilyl, tert.-Butyldimethylsilyl, Benzyliden und Oxomethylen.

Bevorzugte Schutzgruppen der geschützten Substituenten von $R^3$, $R^4$ und $R^5$ sind identisch mit den vorstehend genannten.

Die Produkte (I) und (II) bilden eine große Vielzahl pharmakologisch annehmbarer Salze mit anorganischen und organischen Basen. Diese umfassen beispielsweise Metallsalze, die sich von Alkalimetall- oder Erdalkalimetallhydroxiden, -carbonaten oder -bicarbonaten ableiten, und Salze, die sich von primären, sekundären oder tertiären Aminen ableiten, wie Monoalkylamine, Dialkylamine, Trialkylamine, niedrig-Alkanolamine, Di-niedrig-alkanolamine, niedrig-Alkylendiamine, N,N-Diaralkyl-niedrig-alkylendiamine, Aralkylamine, Amino-subst.-niedrig-alkanole, N,N-Di-niedrigalkylamino-subst.-niedrig-alkanole, Amino-, Polyamino- und Guanidino-subst.-niedrig-Alkansäuren und Stickstoff enthaltende heterocyclische Amine. Beispiele für Salze sind solche, die sich von Natriumhydroxid, Natriumcarbonat, Natriumbicarbonat, Kaliumcarbonat, Kaliumhydroxid, Calciumcarbonat, Trimethylamin, Triethylamin, Piperidin, Morpholin, Chinin, Lysin, Protamin, Arginin, Procain, Ethanolamin, Morphin, Benzylamin, Ethylendiamin, N,N′-Dibenzylethylendiamin, Diethanolamin, Piperazin, Dimethyl-aminoethanol, 2-Amino-2-methyl-1-propanol, Theophyllin, N-Methylglucamin u.ä. ableiten.

Gegenstand der erfindungsgemäßen Verwendung sind weiterhin Salze von Aminogruppen, die in bestimmten Species I und II an den Seitenketten von $R^3$, $R^4$ und $R^5$ enthalten sind. Solche pharmazeutisch annehmbaren Säureadditionssalze leiten sich von organischen und anorganischen Säuren, wie HCl, HBr,

Citronensäure, Weinsäure u.ä., ab.

Die Salze können Monosalze, wie das Monoatriumsalz, das durch Behandlung von 1 Äquiv. Natriumhydroxid mit 1 Äquiv. der Produkte (I) und (II) erhalten wird, wie auch gemischte Disalze sein. Solche Salze können durch Behandlung von 1 Äquiv. einer Base mit einem zweiwertigen Kation, wie Calciumhydroxid, mit 1 Äquiv. der Produkte (I) und (II) erhalten werden. Diese Salze sind pharmakologisch annehmbare, nichttoxische Derivate, die als aktiver Bestandteil in geeigneten pharmazeutischen Dosiseinheitsformen verwendet werden können. Sie können ebenfalls mit anderen Arzneimitteln unter Bildung von Zubereitungen mit einem breiten Aktivitätsspektrum kombiniert werden.

Diese stabilen Oxapen-2-em-carbonsäuren sind wertvolle antimikrobielle Substanzen, die gegenüber verschiedenen grampositiven und gramnegativen Pathogenen wirksam sind. Die freie Säure und insbesondere ihre Salze, wie die Amin- und Metallsalze, insbesondere die Alkalimetall, und Erdalkalimetallsalze, sind nützliche Bakterizide und können zur Entfernung empfindlicher Pathogene von zahnmedizinischen und medizinischen Vorrichtungen, für die Abtrennung von Mikroorganismen und für die therapeutische Verwendung bei Menschen und Tieren eingesetzt werden. Zu diesem letzteren Zweck werden pharmakologisch annehmbare Salze mit anorganischen und organischen Basen, wie sie an sich bekannt sind und bei der Verabreichung von Penicillinen und Cephalosporinen verwendet werden, verwendet. Beispielsweise können Salze, wie Alkalimetall- und Erdalkalimetallsalze, und primäre, sekundäre und tert. Aminsalze für diesen Zweck verwendet werden. Diese Salze können mit pharmazeutisch annehmbaren flüssigen und festen Trägern unter Bildung geeigneter Dosiseinheitsformen wie Pillen, Tabletten, Kapseln, Suppositorien, Sirupen, Elixieren u.ä., verwendet werden, die nach an sich bekannten Verfahren hergestellt werden können.

Die Verbindungen sind wertvolle Antibiotika gegenüber verschiedenen grampositiven und gramnegativen Bakterien und finden dementsprechend in der Human- und Veterinärmedizin Verwendung. Die Verbindungen können als antibakterielle Arzneimittel zur Behandlung von Infektionen verwendet werden, die durch grampositive oder gramnegative Bakterien erzeugt werden, z.B. gegen Staphylococcus aureus, Escherichia coli, Klebsiella pneumoniae, Bacillus subtilis, Salmonella typhosa, Pseudomonas und Bacterium proteus.

Die antibakteriellen Mittel können weiterhin als Zusatzstoffe für Tierfutter, für die Konservierung von Nahrungsmitteln bzw. Futter und als Desinfektionsmittel verwendet werden. Beispielsweise können sie in wäßrigen Zubereitungen in Konzentrationen im Bereich von 0,1 bis 100 Teilen Antibiotikum/Million Teile Lösung zur Zerstörung und Inhibierung des Wachstums schädlicher Bakterien auf medizinischen und zahnmedizinischen Geräten und als Bakterizide bei industriellen Anwendungen, z.B. bei Anstrichmitteln auf Wassergrundlage und in weichem Wasser der Papiermühlen, zur Inhibierung des Wachstums schädlicher Bakterien verwendet werden.

Die Produkte können allein oder zusammen als aktiver Bestandteil in irgendeiner Vielzahl von pharmazeutischen Zubereitungen verwendet werden. Diese Antibiotika und ihre entsprechenden Salze können in Kapselform oder als Tabletten, Pulver oder flüssige Lösungen oder als Suspensionen oder Elixiere verwendet werden. Sie können oral, intravenös oder intramuskulär verabreicht werden.

Die Zubereitungen werden bevorzugt in einer für die Absorption durch den gastro-intestinalen Trakt geeigneten Form verabreicht. Tabletten und Kapseln für die orale Verabreichung können in Dosiseinheitsform vorliegen und sie können übliche Arzneimittelträgerstoffe, wie Bindemittel, z.B. Sirup, Akaziengummi, Gelatine, Sorbit, Tragant oder Polyvinylpyrrolidon; Füllstoffe, z.B. Lactose, Zucker, Maisstärke, Calciumphosphat, Sorbit oder Glycerin; Schmiermittel, z.B. Magnesiumstearat, Talk, Polyethylenglykol, Siliciumdioxid; Desintegrationsmittel, z.B. Kartoffelstärke, oder annehmbare Benetzungsmittel, wie Natriumlaurylsulfat, enthalten. Die Tabletten können nach an sich gut bekannten Verfahren beschichtet werden. Orale, flüssige Zubereitungen können in Form von wäßrigen oder öligen Suspensionen, Lösungen, Emulsionen, Sirupen, Elixieren usw. vorliegen, oder sie können als Trockenprodukt, z.B. für die Rekonstitution mit Wasser oder anderen geeigneten Trägern vor der Verwendung, vorliegen. Solche flüssigen Präparationen können an sich bekannte Zusatzstoffe, wie Suspensionsmittel, z.B. Sorbitsirup, Methylcellulose, Glycose/Zuckersirup, Gelatine, Hydroxyethylcellulose, Carboxymethylcellulose, Aluminiumstearatgel, oder hydrierte genießbae Öle, z.B. Mandelöl, fraktioniertes Cocosnußöl, ölige Ester, Propylenglykol oder Ethylalkohol; Konservierungsmittel, z.B. Methyl- oder Propyl-p-hydroxybenzoat oder Sorbinsäure, enthalten. Suppositorien können an sich bekannte Suppositoriengrundstoffe, z.B. Kakaobutter oder andern Glyceride, enthalten.

Die Zubereitungen für die Injektion können in Dosiseinheitsform in Ampullen oder in Behältern mit mehreren Dosen mit einem zugegebenen Konservierungsstoff vorliegen. Die Zubereitungen können in form von Suspensionen, Lösungen oder Emulsionen in öligen oder wäßrigen Trägern vorliegen, und sie können Formulationsmittel, wie Suspensions-, Stabilisations- und/oder Dispersionsmittel enthalten. Alternativ kann der aktive Bestandteil in Pulverform für die Rekonstitution mit einem geeigneten Träger, z.B. sterilem, pyrogenfreiem Wasser, vor der Verwendung vorliegen.

Die Zubereitungen können ebenfalls in einer für die Adsorption durch die Schleimhautmembranen der Nase und des Halses oder der Bronchialgewebe geeigneten Form vorliegen, und sie können zweckdienlich in Form von Pulvern oder flüssigen Sprays oder Inhalationsmitteln, Lutschbonbons, als Auspinselungsmittel für den Hals, etc. vorliegen. Für die Medikation der Augen und Ohren können die Zubereitungen in Form einzelner Kapseln in flüssiger oder semi-fester Form vorliegen, oder sie können als Tropfen usw. verwendet werden. Topische Anwendungen können in hydrophoben oder hydrophilen Grundstoffen als Salben, Cremes, Lotionen, Auspinselungsmittel, Pulver usw. vorliegen bzw. formuliert werden.

Die Zubereitungen können zusätzlich zu dem Träger einen anderen Bestandteil, wie Stabilisatoren, Bindemittel, Antioxydantien, Konservierungsmittel, Schmiermittel, Suspensionsmittel, Viskositätsmittel oder Geschmacksmittel u.ä., enthalten, Zusätzlich können in den Zubereitungen andere aktive Bestandteile enthalten sein, so daß man ein breiteres antibiotisches Aktivitätsspektrum erhält.

Für die Veterinärmedizin können die Zubereitungen z.B. als intramammare Zubereitung in entweder langwirkenden oder schnellfreisetzenden Grundstoffen formuliert werden.

Die zu verabreichende Dosis hängt in großem Ausmaß von dem Zustand des zu behandelnden Subjekts und dem Gewicht des Wirts, dem Weg und der Frequenz der Verabreichung ab. Der parenterale Weg ist für generalisierte Infektionen und der orale Weg für intestinale Infektionen bevorzugt. Im allgemeinen enthält eine tägliche orale Dosis etwa 15 bis etwa 200 mg aktiven Bestandteil/kg Körpergewicht des Subjekts bei einer oder mehreren Anwendungen pro Tag. Eine bevorzugte tägliche Dosis für erwachsene Menschen liegt im Bereich von etwa 40 bis 120 mg aktiven Bestandteil/kg Körpergewicht.

Die Zubereitungen können in verschiedenen Einheitsdosisformen, z.B. in festen oder flüssigen, oral aufnehmbaren Dosisformen, verabreicht werden. Die Zubereitungen pro Einheitsdosis können entweder in fester oder flüssiger Form 0,1 bis 99 % aktives Material enthalten. Der bevorzugte Bereich beträgt etwa 10 bis 60 %. Die Zubereitungen können im allgemeinen 15 bis etwa 1500 mg aktiven Bestandteil enthalten, im allgemeinen ist es jedoch bevorzugt, eine Dosismenge im Bereich von etwa 250 bis 1000 mg zu verwenden. Bei der parenteralen Verabreichung wird die Einheitsdosis normalerweise die reine Verbindung in einer sterilen Wasserlösung sein oder in Form eines löslichen Pulvers, das aufgelöst werden kann, vorliegen.

Bestimmung der $\beta$-Lactamase-Aktivität

Methoden: Der Abbau von Mezlocillin wurde mit dem Agar-Lochtest mit Streptococcus pyogenes W bestimmt, dessen Empfindlichkeitsgrenze unter 1 mcg/ml liegt.

Die Koloniebildungsfähigkeit der Bakterien wurde durch Ausplattieren der Bakterien auf OXOID-Iso-Sensitest-Agar bestimmt. Die beiden Methoden sind internationale Standardmethoden.

Abbau von Mezlocillin durch Betalaktamase und Verhinderung dieses Abbaus durch Oxapenem "O"

| | Mezlocillin in mcg/ml<br>Reaktionszeit (min) | | | |
|---|---|---|---|---|
| | 0 | 30 | 60 | 240 |
| Betalaktamase aus Pseudomonas aeruginosa W: ohne Oxapenem | 100 | 35 | 0 | |
| mit 1 mcg Oxapenem | 100 | 100 | 100 | |
| mit 1 mcg Clavulansäure | 100 | 40 | 0 | |
| " 10 mcg " | 100 | 27 | 0 | |
| Betalaktamase aus E. coli 4787 TEM 1: ohne Oxapenem | 100 | 65 | 43 | 0 |
| mit 10 mcg Oxapenem | 100 | 100 | 100 | |

Synergistische Wirkung der Kombination. Abnahme der Koloniebildungsfähigkeit von Staphylococcus aureus 25 455/1 durch die Einwirkung von Mezlocillin in flüssigem Nährmedium.

| | Zahl der Kolonien nach sechs Stunden Einwirkungszeit (log N) |
|---|---|
| Kontrolle | $3,5 \times 10^8$ |
| 0,1 mcg Oxapenem | $1,4 \times 10^8$ |
| 2,0 mcg Mezlocillin | $1,0 \times 10^6$ |
| 0,1 mcg Clavulansäure | $1,0 \times 10^8$ |
| 2,0 mcg Mezlocillin plus 0,1 mcg Clavulansäure | $5,5 \times 10^4$ |
| 2,0 mcg Mezlocillin plus 0,1 mcg Oxapenem | $4,0 \times 10^2$ |

Die folgenden Beispiele erläutern die Produkte, Verfahren, Zubereitungen und Behandlungsverfahren (nicht erfindungsgemäß).

Beispiel 1

Herstellung von 2-tert.-Butyl-1-oxapen-2-em-3-carbonsäure, dem p-Nitrobenzylester und ihre Natriumsalz

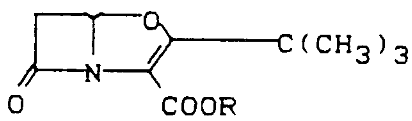

R = H, $CH_2C_6H_4NO_2$, Na Stuffe $A_1$: tert.-Butylthioazetidin-2-on

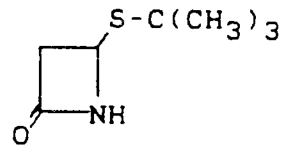

Zu einer Lösung von 9,689 g (75 mMol) 4-Acetoxyazetidin-2-on und 7,76 g (86 mMol) tert.-Butylmercaptan in 75 ml trockenem THF werden bei -3° C unter Rühren 13.13 g Diazabicycloundecen (DBU) innerhalb von 35 Minuten getropft, so daß die Reaktionstemperatur nicht über - 1,5° C steigt. Nach Aufbewahrung über Nacht bei 0° C wird noch 1,5 Stunden bei Raumtemperatur gerührt. Verdünnen mit 500 ml Methylenchlorid, Waschen mit 100 ml gesättigter wäßriger Kochsalzlösung, mit 100 ml 2N Salzsäure und weiteren 100 ml Kochsalzlösung, Trocknen der organischen Phase über Magnesiumsulfat und Eindampfen des Lösungsmittels im Vakuum ergibt einen festen Rückstand, der über 300 g Kieselgel mit Toluol-Essigsäureethylester 2:1 chromatographiert wird. Nach Umkristallisieren des chromatographierten Produktes aus Methylenchlorid-Hexan werden 6,5 g reine Titelverbindung vom Schmp. 119-121° C erhalten. IR-Spektrum in Methylenchlorid: 3410, 2955, 2905, 2865, 1770, 1460, 1370, 1160, 970, 925 $cm^{-1}$.

Alternative Herstellung von tert.-Butylthioazetidin-2-on aus 4-Benzoyloxyazetidin-2-on

Zu einer Lösung von 9.3 ml (82,5 mMol) tert.-Butylmercaptan in 37,5 ml Acetonitril werden bei 0° C 41,25 ml (82,5 mMol) 2N NaOH in Wasser getropft. Innerhalb von 25 Minuten wird dann eine Lösung (Erwärmen) 14,32 g (75 mMol) 4-Benzoyloxyazetidin-2-on in 56 ml Acetonitril zugetropft, so daß die Reaktionstemperatur nicht über 0° C steigt. Der intermediär beim Zutropfen entstandene Niederschlag löst sich vollständig auf beim weiteren Rühren bei 0° C. Das Gemisch wird dann bei 0° C über Nacht stehen gelassen, wobei die Dünnschichtchromatographie auf Kieselgel mit Toluol-Essigsäureethylester (1:1) kein Ausgangsmaterial mehr anzeigt. Die gelbe Reaktionslösung wird mit 500 ml Methylenchlorid versetzt, die wäßrige Phase abgetrennt und nochmals mit 100 ml Methylenchlorid extrahiert. Die vereinigten Extraktionslösungen werden nacheinander gewaschen mit je 100 ml I N HCl-Lösung, zweimal mit $NaHCO_3$-Lösung und einmal mit verdünnter NaCl-Lösung. Trocknen der organischen Phase über $MgSO_4$, Filtration und Eindampfen des Lösungsmittels im Vakuum ergibt 11.8 g (99 %) eines gelben kristallinen Rückstandes. Umkristallisation aus 160 ml Dibutylether bei 90° C → 0° C ergibt 10.3 g (86 %) reine Titelverbindung vom Schmp. 119-120° C.

Stufe B: (4-tert.-Butylthio-2-oxo-1-azetidinyl)-essigsäure-p-nitrobenzylester

Zu einer Lösung von 1.91 g (12mMol) tert.Butylthioazetidin-2-on in 6 ml trockenem N,N-Dimethylformamid (DMF) werden unter Rühren bei -70° C 14,4 ml 1N Lösung von Lithium-bis-trimethylsilylamid in Tetrahydrofuran (THF) getropft und anschließend zum Gemisch eine Lösung von 4.93 g (18mMol) Bromessigsäure-p-nitrobenzylester in 6 ml DMF zugetropft und das Gemisch noch 30 Minuten bei -30° C gerührt. Verdünnen des Reaktionsgemisches mit 100 ml Toluol, Waschen mit drei Portionen von je 50 ml Wasser, Trocknen der organischen Phase über Magnesiumsulfat und Eindampfen des Lösungsmittels in

Vakuum ergibt 4.3 g festes Rohprodukt, welches über 120 g Kieselgel mit Toluol-Essigsäureethylester (4:1) chromatographiert wird. Das gereinigte Produkt (2,6 g) wird aus 100 ml trockenem Isopropanol umkristallisiert. Ausbeute: 2,09 g vom Schmp. 82.5-84 ° C. IR-Spektrum in Methylenchlorid: 2955, 1770, 1755, 1610, 1530, 1390, 1375, 1365, 1345, 1180, 1110, 945, 915, 855, 845 $cm^{-1}$.

Stufe C: 2-(4-tert.-Butylthio-2-oxo-1-azetidinyl)-4,4-dimethyl-3-oxopentansäure-p-nitrobenzylester

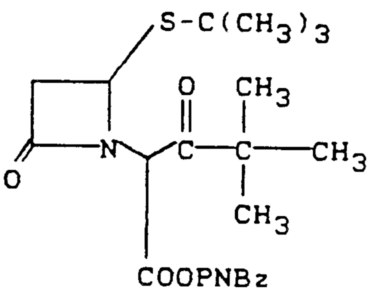

Zu einer Lösung von 1.059 mg (3 mMol) (4-tert.-Butylthio-2-oxo-1-azetidinyl)-essigsäure-p-nitrobenzylester in 7 ml trockenem THF werden bei -70 ° C 6 ml einer frisch hergestellten 1M Lösung von Lithium-bis-trimethylsilylamid in THF zugetropft und anschließend bei -70 ° C eine Lösung von 382 mg Pivaloylchlorid in 1 ml THF zugetropft und das Reaktionsgemisch noch während 30 Minuten bei der gleichen Temperatur gerührt. Das Gemisch mit 200 ml Toluol verdünnt und mit wenig wäßriger Essigsäure versetzt. Waschen der organischen Phase mit 100 ml 2N wäßriger Salzsäure und zweimaliges Waschen mit gesättigter Kochsalzlösung (100 ml), Trocknen der organischen Phase mit $MgSO_4$ und Eindampfen des Lösungsmittels im Vakuum ergibt ein dunkelrotes Öl. Die Reinigung des Rohkproduktes über 40 g Kieselgel mit Toluol-Essigester (9:1) ergibt 795 mg eines nichtkristallinen Festkörpers. IR-Spektrum in Methylenchlorid: 2970, 1770, 1760, 1715, 1610, 1530, 1350, 1315, 1180, 995, 845 $cm^{-1}$.

Stufe $D_1$: 2-(4-Chlor-2-oxo-1-azetidinyl)-4,4-dimethyl-3-oxopentansäure-p-nitrobenzylester

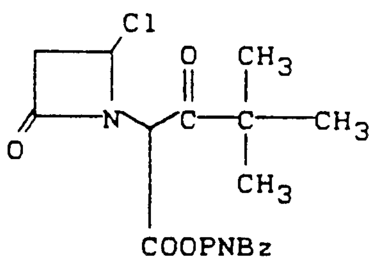

Eine Lösung von 439 mg (1.0 mMol) 2-(4-tert.-Butylthio-2-oxo-1-azetidinyl)-4,4-dimethyl-3-oxopentansäure-p-nitrobenzylester in 20 ml trockenem Methylenchlorid wird auf -50 ° C gekühlt und mit einer Lösung von 166 mg Chlor in 1,6 ml Tetrachlorkohlenstoff versetzt. Nach 30 minütigem Rühren bei -50 ° C wird das Lösungsmittel im Vakuum eingedampft und der Rückstand aus Methylenchlorid-Hexan umkristallisiert, wobei das Produkt (348 mg) als kristalliner Festkörper als 6:4 Gemisch der beiden diastereomeren Titelverbindungen erhalten wird.
Smp. 96-100.5 ° C, Zersetzung. $^1$H-NMR ($CD_3CN$): $\delta$ = 1.04 (s,~ 5.4 H, t-Butyl I), 1.21 (s, ~ 3.6 H), t-Butyl II), 3.05-3.86 (m, 1H, 3'-H), 5.29 (s, 2 H, -O-$CH_2$Ar), 5.52 (s, ~ 0,6 H, 2-H, I), 5.71 (s, ~ 0,4, 2-H, II), 5.84 (dd, J=2 Hz, J= 2 Hz, J=4 Hz ~ 0,6 H, 4'-H, I), 5.98 (dd, J=2 Hz, J = 4 Hz, ~ 0,4 H, 4'-H, II) 7,51 (d, J = 9 Hz, ~ 0,8 H, Ar-H, II), 7,55 (d, J = 9 Hz, ~ 1,2 H, Ar-H, I), 8.19 (d, J = 9 Hz, 2 H, Ar-H, I und II).

Stufe $D_2$: 3-tert.-Butyl-7-oxo-4-oxa-1-azabicyclo[3.2.0]-hept-2-en-2-carbonsäure-p-nitrobenzylester

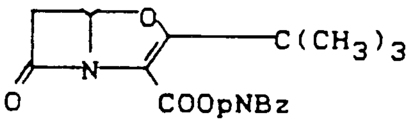

Das Gemisch der diastereomeren 2-(Chlor-2-oxo-1-azetidinyl)-4,4-dimethyl-3-oxopentansäure-p-nitrobenzylester (348 mg, 0.91 mMol) wurde in trockenem THF (10 ml) gelöst und bei 0° C mit, 0,91 ml einer frisch zubereiteten 1 M Lösung von Kalium-tert.-butylat in tert.-Butanol versetzt und das Reaktionsgemisch bei 0° C während 15 Minuten gerührt. Verdünnen mit 150 ml Benzol, dreimaliges Waschen mit je 50 ml 0,5 M Phosphatpufferlösung pH = 7, Trocknen der organischen Phase über $MgSO_4$ und Eindampfen des Lösungsmittels im Vakuum liefert einen fahlgelben Festkörper, der über 9 g Kieselgel mit Benzol-Essigsäureethylester 97:3 chromatographiert wird, wobei 237 mg Produkt erhalten werden. Umkristallisation aus Methylenchlorid-Hexan liefert 200 mg blaßgelbe Kristalle vom Schmp. 142-144° C.
$^1$H-NMR($CD_3CN$): $\delta$ = 1.29 (s, 9 H, tert.-Butyl), 3,40 (dd, J = 17 Hz, J = 1 Hz, 1 H, 6-H transständig), 3.79 (dd, J = 17 Hz, J = 2,5 Hz, 1 H, 6-H cisständig), 5.16 (d, J = 14 Hz, 1H, -O-$CH_2$-Ar), 5.42 (d, J = 14 Hz, 1 H, -O-$CH_2$-Ar), 5.85 (dd, J = 2,5 Hz, J = 1 Hz, 1 H, 5-H), 7.61 (d, J = 8,5 Hz, 2 H, Ar-H), 8.17 (d, J = 8,5 Hz, 2 H, Ar-H). IR-Spektrum in Methylenchlorid: 2955, 1804, 1715, 1610, 1585, 1525, 1350, 1315, 1200, 1165, 1145, 1120, 1080, 1040, 1025, 1015, 885, 855, 840 $cm^{-1}$. UV-Spektrum in Dioxan: $\lambda_{max}$ = 277 nm ($\epsilon$ = 15340).

Stufe E: 3-tert.-Butyl-7-oxo-1-azabicyclo[3.2.0]-hept-2-en-2-carbonsäure, Na-Salz

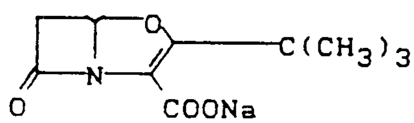

Zu einem auf 0° C gekühltem Gemisch von 30 mg Palladium auf Kohle (10 %), 2 ml Essigsäureethylester und einer Lösung von 4,7 mg (56 $\mu$mol) Natriumhydrogencarbonat in 1 ml Wasser in einer Wasserstoffatmosphäre wird über ein Septum eine Lösung von 17,3 mg (50 $\mu$mol) 3-tert.-butyl-7-oxo-4-oxa-1-azabicyclo-[3.2.0]-hept-2-en-2-carbonsäure-p-nitrobenzylester in 1 ml Essigsäureethylester eingesetzt und hydriert. Innerhalb von 20 Minuten werden 5,4 ml Wasserstoff verbraucht, etwas mehr als die theoretisch erforderliche Menge (4,6 ml). Das mehrphasige Gemisch wird filtriert unter Kühlung und das gekühlte (0° C) Filtrat zweimal mit je 3 ml Essigsäureethylester gewaschen. Die wäßrige Lösung wird sofort lyophilisiert im Hochvakuum, wobei 8,8 mg eines weißen Festkörpers erhalten werden: UV ($H_2O$): $\lambda_{max}$ = 269 nm ($\epsilon$ = 5800) 360 MHz-$^1$H-NMR-Spektrum in $D_2O$: $\delta$ = 1.23 (s, 9 H, tert.-Butyl), 3.43 (dd, J = 18 Hz, J = 1 Hz, 1 H, 6-H transständig), 3.72 (dd, J = 18 Hz, J = 2,5 Hz, 1 H, 6-H cisständig), 5.82 (s, 1H, 5-H).

Beispiel 2

Herstellung von 2-tert.-Butyl-6-methyl-1-oxapen-2-em-3-carbonsäure, dem p-Nitrobenzylester und ihrem Natriumsalz

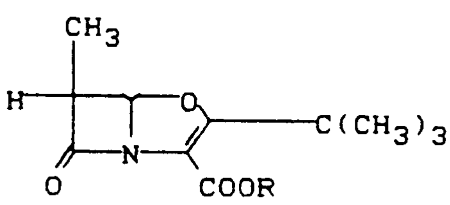

R = H, Na, p-NBz

R = H, Na, p-NBz

Nach dem in Beispiel 1 beschriebenen Verfahren unter Verwendung der gleichen Reaktionsbedingungen wurde ausgehend von 4-Acetoxy-3-methylazetidin-2-on über die Stufen $A_1$, B und C 2-(4-tert.-Butylthio-3-methyl-2-oxo-1-azetidinyl)-4,4-dimethyl-3-oxopentansäure-p-nitrobenzylester

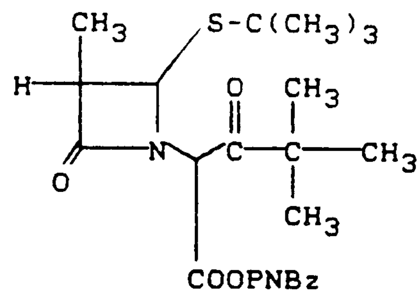

als nichtkristalliner Festkörper erhalten. IR-Spektrum in Methylenchlorid: 2955, 1765, 1760, 1720, 1610, 1525, 1460, 1380, 1365, 1350, 1315, 1205, 1180, 1120, 1050, 855, 840 $cm^{-1}$, UV-Spektrum in Ethanol: $\lambda_{max}$ = 264 nm ($\epsilon$ = 10160).

Stufe $D_1$: 2-(4-Chlor-3-methyl-2-oxo-1-azetidinyl)-4,4-dimethyl-3-oxopentansäure-p-nitrobenzylester

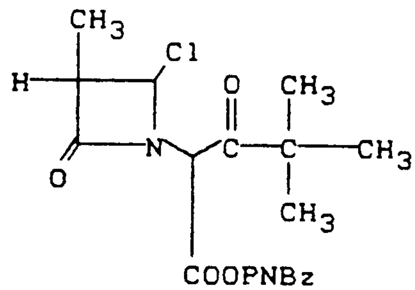

Nach dem im Beispiel 1 beschriebenen Verfahren unter Verwendung der gleichen Reaktionsbedingungen wurde ausgehend von 2-(4-tert.-butylthio-3-methyl-2-oxo-1-azetidinyl)-4,4-dimethyl-3-oxopentansäure-p-nitrobenzylester die Titelverbindung (Stufe $D_1$) als nichtkristalliner Festkörper (Gemisch von zwei Diastereomeren) erhalten. NMR-Spektrum in $CD_3CN$: $\delta$ = 1.19, 1.21 und 1.32 (3 Signale, 12 H), 3.59-3.98 (m, 1 H), 5.30 (s, 2H), 5.50 (s, ~ 0.25 H), 5.70 (s, ~ 0,75 H), 5.94 d, J = 5 Hz. ~ 0,25 H), 6.09 (d, J = 5 Hz, ~ 0,75 H), 7.43 - 7.64 (m, 2H), 8.17 (d, J = 9 Hz, 2 H).

Stufe $D_2$: 2-tert.-Butyl-6-methyl-1-oxapen-2-em-3-carbonsäure-p-nitrobenzylester (3-tert.-Butyl-7-oxo-4-oxa-1-azabicyclo[3.2.0]hept-2-en-2-carbonsäure-p-nitrobenzylester)

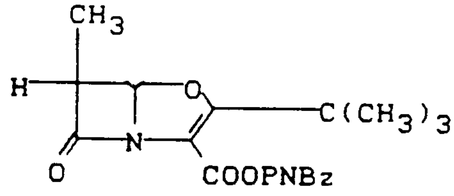

Nach dem im Beispiel 1 beschriebenen Verfahren unter Verwendung der gleichen Reaktionsbedingungen wurde ausgehend von 2-(4-Chlor-3-methyl-2-oxo-1-azetidinyl)-4,4-dimethyl-3-oxopentansäure-p-nitrobenzylester die Titelverbindung als nichtkristalliner Festkörper (Gemisch von cis-trans Isomeren) erhalten.

IR-Spektrum in $CH_2Cl_2$: 2965, 1700, 1715, 1585, 1525, 1345, 1310, 1165, 1140, 1085, 1025, 1015, 935, 850 $cm^{-1}$. UV-Spektrum in Dioxan: $\lambda_{max}$ = 277 nm ($\epsilon$ = 15200).

Stufe E: 3-tert.-Butyl-6-methyl-7-oxo-4-oxa-1-azabicyclo[3.2.0]-hept-2-en-2-carbonsäure, Na-Salz

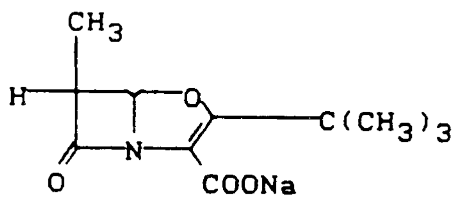

Nach dem im Beispiel 1 beschriebenen Verfahren unter Verwendung der gleichen Reaktionsbedingung wurde ausgehend von 3-t-butyl-6-methyl-7-oxo-4-oxa-1-azabicyclo[3.2.0]hept-2-en-2-carbonsäure-p-nitrobenzylester die Titelverbindung als weißer Festkörper (Lyophilisat) erhalten. UV-Spektrum in $H_2O$: $\lambda_{max}$ = 260 nm (c = 5800). $^1$H-NMR-Spektrum in $D_2O$: 1.24 und 1.27 (2s, 9H), 1.38 (d, J = 7,5 Hz), 3.67 (q, J = 7,5 Hz, ~ 0,5 H, trans), 3.96 (dq, J = 7,5 Hz, ~ J = 3 Hz, ~ 0,5 H, cis). 5.55 (s, ~ 0,5 H, trans), 5.80 (d, J = 3 Hz, ~ 0,5 Hz, cis).

Beispiel 3

Herstellung von 2-tert.-Butyl-6,6-dimethyl-1-oxapen-2-em-3-carbonsäure, dem p-Nitrobenzylester und ihrem Natriumsalz

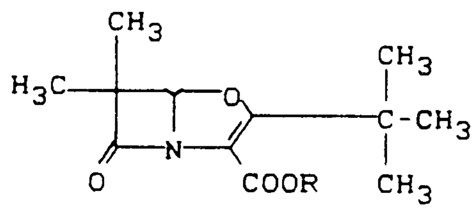

R = H, Na, pNBz

Nach dem im Beispiel 1 beschriebenen Verfahren unter Verwendung der gleichen Reaktionsbedingungen wurde ausgehend von 4-Acetoxy-3,3-dimethylazetidin-2-on über die Stufen $A_1$, B und C 2-(4-tert.-Butylthio-3,3-di-methyl-2-oxo-1-azetidinyl)-4,4-dimethyl-3-oxopentansäure-p-nitrobenzylester als kristalliner Festkörper vom Schmp. 87,5-90,5° C aus

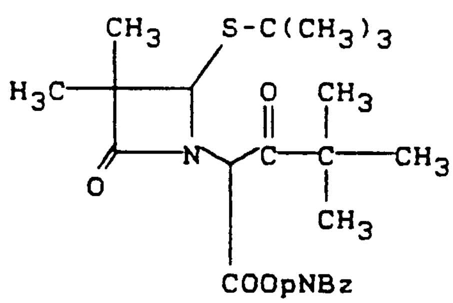

Methylenchlorid-Hexan (Gemisch von zwei Diastereomeren) erhalten. IR-Spektrum in Methylenchlorid: 2955, 2865, 1765, 1755, 1715, 1610, 1525, 1460, 1390, 1370, 1350, 1315, 1185, 1135, 1105, 995, 850 $cm^{-1}$. UV-Spektrum in Ethanol:

$\lambda_{max}$ = 264.5 nm ($\epsilon$ = 10930).

Stufe D: 3-tert.-Butyl-6,6-dimethyl-7-oxo-4-oxa-1-azabicyclo[3.2.0]hept-2-en-2-carbonsäure-p-nitrobenzylester

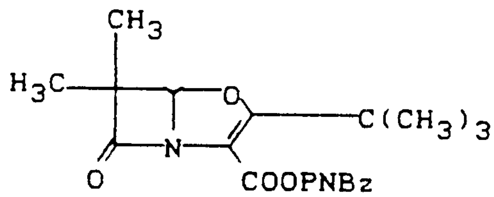

Eine Lösung von 930 mg (2.0 mMol) 2-(4-tert.-Butylthio-3,3-dimethyl-2-oxo-1-azetidinyl)-4,4-dimethyl-3-oxo-pentansäure-p-nitrobenzylesterin 460 ml trockenem Dimethoxyethan wurde zusammen mit 1046 mg (5.0 mMol) gelbem Quecksilber(II)chlorid kräftig gerührt und am Rückfluß drei Stunden gekocht. Nach dem Abkühlen wurde die gelbliche Lösung durch Celite filtriert und auf etwa ein Zehntel des Volumens eingeengt. Nach Verdünnung mit 500 ml Benzol und Stehenlassen für zwei Tage bei 0° C wurde von dem entstandenen farblosen Niederschlag abfiltriert und die erhaltene klare Lösung mit 250 ml gesättigter Kochsalzlösung, 250 ml 0,5 M Phosphatpufferlösung pH 7 und 250 ml gesättigter Kochsalz-lösung gewaschen. Trocknung der organischen Phase mit Magnesiumsulfat, Einengen der Lösung auf 50 ml und Stehenlassen bei 0° C, Abfiltrieren von wenig erneut angefallenem Niederschlag und Abziehen des Lösungsmittels im Vakuum ergaben ein gelbes, leicht getrübtes Öl. Chromatographie des Rohproduktes über 25 g Florisil mit Benzol-Essigsäureethylester (7:1) lieferte 560 mg reine Titelverbindung. Nach Umkristallisieren aus Methylenchlorid-Hexan beträgt der Schmp, 119-120.5° C.

IR-Spektrum in Methylenchlorid: 2935, 2870, 1797, 1715, 1610, 1585, 1525, 1460, 1350, 1315, 1155, 1140, 1085, 1010, 850 $cm^{-1}$, UV-Spektrum in Dioxan: $\lambda_{max}(\epsilon$ = 278 nm ($\epsilon$ = 14980). Massenspektrum (20eV, 80° C): 374 $M^+$. Von dieser Substanz wurde eine Röntgenstrukturanalyse durchgeführt, welche die Struktur bestätigt.

Stufe E: 3-tert-Butyl-6,6-dimethyl-7-oxo-4-oxa-1-azabicyclo[3.2.0]hept-2-en-2-carbonsäure, Na-Salz

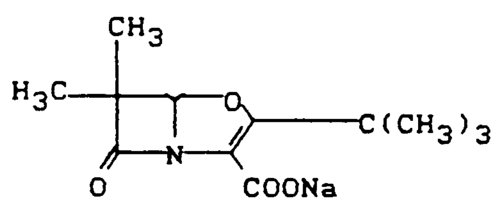

Nach dem im Beispiel 1 beschriebenen Verfahren unter Verwendung der gleichen Reaktionsbedingungen wurde ausgehend von 3-tert.-Butyl-6,6-dimethyl-7-oxo-3-oxa-1-azabicyclo[3.2.0]hept-2-en-2-carbonsäure-p-nitrobenzylester die Titelverbindung als blaßgelber Festkörper (Lyophilisat) erhalten. UV-Spektrum in $H_2O$: $\lambda_{max}$ = 261 nm. NMR-Spektrum in $D_2O$: 1.23 (s, 9H), 1.26 (s, 3H), 1.39 (s, 3H), 5.50 (s, 1H).

Beispiel 4

Herstellung von 2-(2-Chlor-1,1-dimethylethyl)-6,6-dimethyl-1-oxapen-2-em-3-carbonsäure, dem p-Nitrobenzylester und ihrem Na-Salz

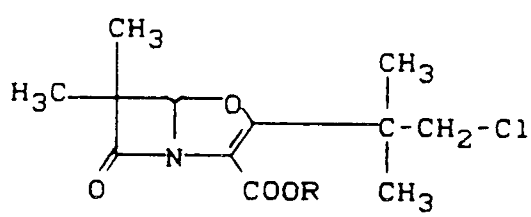

R = H, Na, pNBz

Nach dem im Beispiel 3 beschriebenen Verfahren bei Verwendung der gleichen Reaktionsbedingungen wurde ausgehend von 4-Acetoxy-3,3-dimethylazetidin-2-on unter Verwendung von Chlorpivaloylchlorid in Stufe C über die Stufen $A_1$, B, C, D die Verbindung

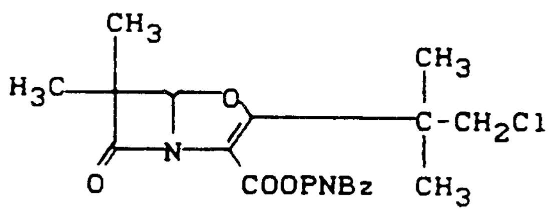

als nichtkristalliner Festkörper erhalten. IR-Spektrum in Methylenchlorid: 2930, 2875, 1803, 1710, 1590, 1525, 1460, 1370, 1350, 1315, 1255, 1160, 1130, 1115, 1090, 1010, 990, 920, 850 $cm^{-1}$.

Stufe E: 3-(2-Chlor-1,1-dimethylethyl)-6,6-dimethyl-7-oxo-4-oxa-1-azabicyclo[3.2.0]hept-2-en-2-carbonsäure, Na-Salz

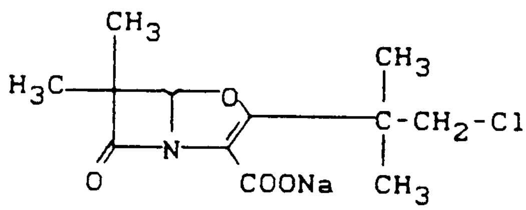

Ausgehend von dem entsprechenden p-Nitrobenzylester wurde nach dem in Beispiel 3 gegebenen Verfahren die Titelverbindung als nichtkristalliner farbloser Festkörper nach Lyophilisation in 60 % Ausbeute erhalten. NMR-Spektrum in $D_2O$: $\delta$ = 1.27, 1.30, 1.33 und 1.39 (4s, 12H), 3.74 (d, J = 10 Hz, 1 H), 4.05 (d. J = 10 Hz, 1 H), 5.52 (s, 1 H). UV-Spektrum in $H_2O$: $\lambda_{max}$ = 265 nm ($\epsilon$ = 5800).

## Beispiel 5

Herstellung von 6,6-Dimethyl-3-(1-methyl-1-phenylethyl)-7-oxo-4-oxa-1-azabicyclo[3.2.0]hept-2-en-2-carbonsäure, ihrem Na-Salz und ihrem p-Nitrobenzylester

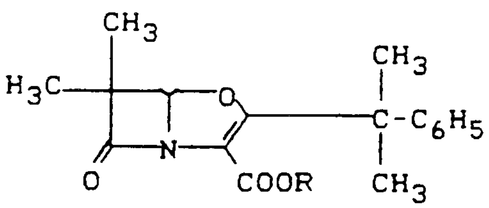

R = H, Na, pNBz

Ausgehend von 2-(4-tert.-butylthio-3,3-dimethyl-2-oxo-azetidinyl)-essigsäure-p-nitrobenzylester und 2-Methyl-2-phenylpropionsäurechlorid wurde nach dem in Beispiel 1 beschriebenen Verfahren über die Stufen C, $D_1$ und $D_2$ die Titelverbindung (p-Nitrobenzylester) als nichtkristalliner leicht gelblicher Festkörper erhalten. IR-Spektrum in $CH_2Cl_2$; 2930, 2875, 1800, 1720, 1600, 1525, 1350, 1320, 1145, 1085, 1075 $cm^{-1}$.

Stufe E: 6,6-Dimethyl-3-(1-methyl-1-phenylethyl)-7-oxo-4-oxa-1-azabicyclo[3.2.0]hept-2-en-2-carbonsäure, Na-Salz

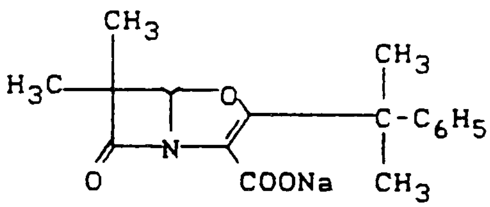

Ausgehend von dem entsprechenden p = Nitrobenzylester wurde nach dem in Beispiel 1 beschriebenen Verfahren die Titelverbindung als farbloser Festkörper nach Lyophilisation in 50 % Ausbeute erhalten. UV-Spektrum in $H_2O$: $\lambda_{max}$ = 263 nm ($\epsilon$ = 5600).

Beispiel 6

Herstellung von 6,6-Dimethyl-3-(1,1-diphenylethyl)-7-oxo-4-oxa-1-azabicyclo[3.2.0]hept-2-en-carbonsäure, ihrem Na-Salz und ihrem p-Nitrobenzylester

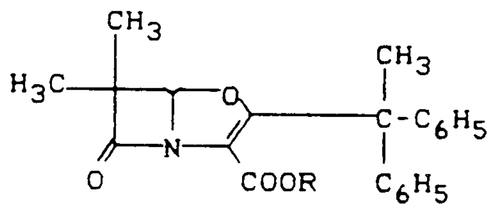

R = H, Na, pNBz

Ausgehend von 2-(4-tert.-butylthio-3,3-dimethyl-2-oxo-azetidinyl)-essigsäure-p-nitrobenzylester und 2,2-Diphenylpropionsäurechlorid wurde nach dem in Beispiel 1 beschriebenen Verfahren über die Stufen C, $D_1$ und $D_2$ die Titelverbindung (p-Nitrobenzylester) als farbloser Festkörper erhalten. IR-Spektrum in $CH_2Cl_2$: 2930, 2875, 1805, 1725, 1600, 1525, 1350, 1315, 1150, 1085, 1075 $cm^{-1}$.

Stufe E: 6,6-Dimethyl-3-(1,1-diphenylethyl)-7-oxo-4-oxa-1-azabicyclo[3.2.0]hept-2-en-2-carbonsäure, Na-Salz

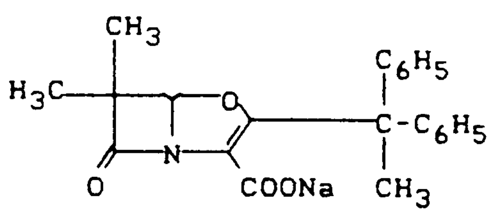

Ausgehend von dem entsprechenden p-Nitrobenzylester wurde nach dem im Beispiel 1 beschriebenen Verfahren die Titelverbindung als farbloser Festkörper nach Lyophilisation in 63 % Ausbeute erhalten. UV-Spektrum in $H_2O$: $\lambda_{max}$ = 265 nm ($\epsilon$ = 6000).

Beispiel 7

Herstellung von 6,6-Dimethyl-3-[1-methyl-1-(2-thienyl)ethyl]-7-oxo-4-oxa-1-azabicyclo[3.2.0]hept-2-en-2-carbonsäure, ihrem Na-Salz und ihrem p-Nitrobenzylester

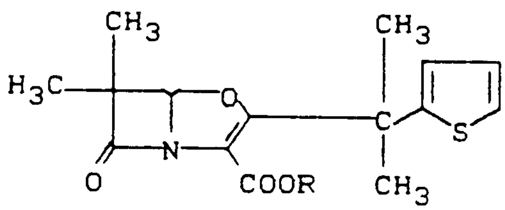

R = H, Na, pNBz

Ausgehend von 2-(4-tert.-butylthio-3,3-dimethyl-2-oxo-azetidinyl)-essigsäure-p-nitrobenzylester und 2-Methyl-2-thienylpropionsäurechlorid wurde nach dem im Beispiel 1 beschriebenen Verfahren über die Stufen C, $D_1$ und $D_2$ die Titelverbindung (p-Nitrobenzylester) als leicht gelblicher Festkörper erhalten. IR-Spektrum in $CH_2Cl_2$: 2930, 1795, 1715, 1590, 1520, 1350, 1310, 1140, 1080 $cm^{-1}$.

Stufe E: 6,6-Dimethyl-3-[1-Methyl-1-(2-thienyl)ethyl-]-7-oxo-4-oxa-1-azabicyclo[3.2.0]hept-2-en-2-carbonsäure, Na-Salz

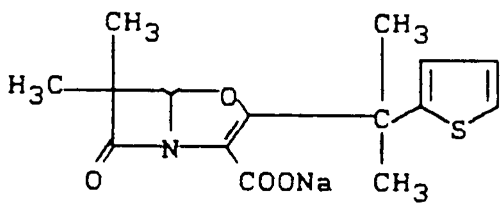

Ausgehend von dem entsprechenden p-Nitrobenzylester wurde nach dem in Beispiel 1 beschriebenen Verfahren die Titelverbindung als farbloser nichtkristalliner Festkörper (Lyophilisat) in 70 % Ausbeute erhalten. UV-Spektrum in $H_2O$: $\lambda_{max}$ = 270 nm ($\epsilon$ = 6500).

Beispiel 8

Herstellung von 3-(2-Amino-1,1-dimethylethyl)-7-oxo-4-oxa-1-azabicyclo[3.2.0]hept-2-en-2-carbonsäure

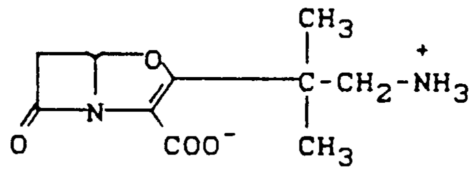

Stufe C: 2-(4-tert.-Butylthio-2-oxo-1-azetidinyl)-5-chlor-4,4-dimethyl-3-oxopentansäure-p-nitrobenzylester

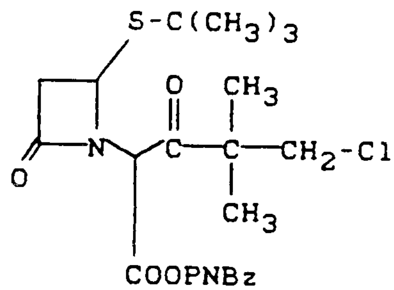

Zu einem Gemisch von 1.06 g (3mMol) 2-(4-tert.-Butylthio-2-oxo-1-azetidinyl)-essigsäure-p-nitrobenzylester und 410 µl (3.17 mMol) Chlorpivaloylchlorid in 38 ml absolutem Tetrahydrofuran wird unter Rühren bei -70° C 6 ml einer 1 M Lösung von Lithium-bis(trimethylsilylamid) in THF langsam zugetropft und das Gemisch noch 30 Minuten bei -70° C gerührt. Das Reaktionsgemisch wird verdünnt mit 250 ml Toluol, mit 10 ml 2 N wäßriger HCl und 100 ml gesättigter NaCl-Lösung versetzt. Nach Abtrennung wird die organische Phase nochmals mit 100 ml gesättigter NaCl-Lösung gewaschen, dann über $MgSO_4$ getrocknet, filtriert und das Lösungsmittel am Vakuum-Rotationsverdampfer entfernt. Der nichtkristalline Rückstand wird über 46 g Kieselgel mit Toluol-Essigsäureethylester (19:1) chromatographiert, wobei 980 mg nichtkristalline Titelverbindung erhalten werden. IR in $CH_2Cl_2$:2930, 1770, 1760, 1725, 1615, 1530, 1465, 1370, 1250, 1215, 1190, 1110, 1040, 1000, 847 $cm^{-1}$.

Umwandlung einer Gruppe $R^3$; 5-Azido-2-(4-tert.-butylthio-2-oxo-1-azetidinyl)-4,4-dimethyl-3-oxopentansäure-p-nitrobenzylester

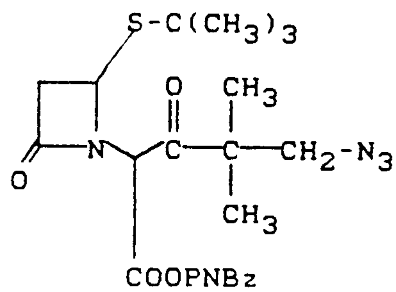

Ein Gemisch von 236 mg (0,5 mMol) 2-(4-tert.-Butylthio-2-oxo-1-azetidinyl)-5-chlor-4,4-dimethyl-3-oxopentansäure-p-nitrobenzylester und 200 mg (1.04 mMol) Triton B-azid in 0,3 ml DMF wird während 20 Stunden bei Raumtemperatur gerührt, dann mit Toluol verdünnt und zweimal mit Wasser gewaschen. Die wäßrige Phase wird mit wenig Toluol extrahiert und die vereinigten organischen Phasen über $MgSO_4$

getrocknet. Filtration und Eindampfen des Lösungsmittels im Vakuum liefert 290 mg eines farblosen Rückstands. IR-Spektrum in $CH_2Cl_2$: 2930, 2860, 2110, 1775, 1755, 1720, 1610, 1530, 1350, 1180, 1120, 850 $cm^{-1}$.

Stufe $D_1$: 5-Azido-2-(4-chlor-2-oxo-1-azetidinyl)-4,4-dimethyl-3-oxopentansäure-p-nitrobenzylester

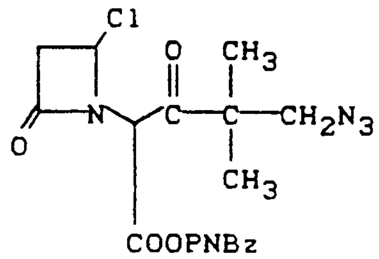

Zu einer Lösung von 110 mg 5-Azido-2-(4-tert.-Butylthio-2-oxo-1-azetidinyl)-4,4-dimethyl-3-oxopentansäure-p-nitrobenzylester in 9 ml Methylenchlorid wird bei -60° C 286 µl einer 1.14 g/10 ml enthaltenden Lösung von Chlor in Tetrachlorkohlenstoff zugegeben und das Gemisch anschließend während zwei Stunden bei -60° C gerührt. Die Reaktionslösung wird eingedampft im Rotationsverdampfer wobei 119 mg gelbes Öl erhalten werden. IR-Spektrum in $CH_2Cl_2$: 2930, 2860, 2105, 1780, 1755, 1720, 1610, 1530, 1350, 1190 $cm^{-1}$.

Stufe $D_2$: 3-(2-Azido-1,1-dimethylethyl)-7-oxo-4-oxa-1-azabicyclo[3.2.0]hept-2-en-2-carbonsäure-p-nitrobenzylester

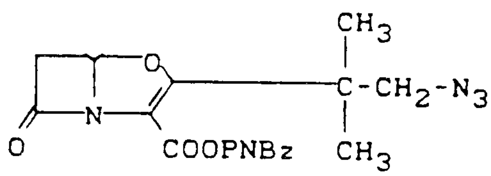

Eine Lösung von 107 mg (0.23 mMol) 5-Azido-2-(4-chlor-2-oxo-1-azetidinyl)-4,4-dimethyl-3-oxopentansäure-p-nitrobenzylester in 4,5 ml trockenem Tetrahydrofuran wird bei -30° C mit 313 µl einer 0,75 M Lösung von Kalium-tert.-butylat in tert.-Butanol versetzt und die Lösung während 30 Min. bei -30° C gerührt. Das Reaktionsgemisch wird dann verdünnt mit 20 ml Essigsäureethylester, und mit 10 ml Wasser und 10 ml NaCl-Lösung gewaschen. Die wäßrigen Phasen werden mit 10 ml Essigsäureethylester extrahiert und die vereinigten organischen Phasen über $MgSO_4$ getrocknet, filtriert und dann im Vakuum eingedampft.

Dabei werden 105 mg eines gelben Rückstandes erhalten, der aus Methylenchlorid-Diisopropylether kristallisiert wird. Ausbeute 61 mg vom Schmelzpunkt 81-82° C. IR-Spektrum in $CH_2Cl_2$: 2950, 2860, 2110, 1810, 1720, 1590, 1530, 1370, 1320, 1085, 1020 $cm^{-1}$.

Stufe E: 3-(2-Amino-1,1-dimethylethyl)-7-oxo-4-oxa-2-azabicyclo[3.2.0]hept-2-en-2-carbonsäure

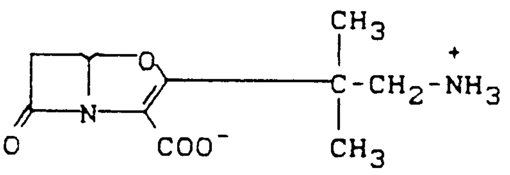

Zu einem vorhydrierten Gemisch von 60 mg Palladium auf Kohle (10 %) in 1 ml Essigsäureethylester und 0.7 ml Wasser wird bei 0° C durch ein Septum eine Lösung von 21 mg (0,054 mMol) 3-(2-Azido-1,1-dimethylethyl)-7-oxo-4-oxa-1-azabicyclo[3.2.0]hept-2-en-2-carbonsäure-p-nitrobenzylester in 1 ml Essigsäureethylester mit einer Spritze zugegeben. Nach 20 Minuten Reaktionszeit werden 5.7 ml Wasserstoff aufgenommen (theoretische Menge: 4.9 ml). Das Reaktionsgemisch wird bei 0° C filtriert und die wäßrige Phase zweimal mit 2 ml vorgekühltem Essigsäureethylester gewaschen. Die wäßrige Phase enthält 8.96 mg der Titelverbindung. UV-Spektrum in $H_2O$: $\lambda_{max}$ = 271 nm ($\epsilon$ = 5000).

Beispiel 9

Herstellung von 3-[1,1-Dimethyl-2-((1-methyl-1,2,3,4-tetrazol-5-yl)-thio)-ethyl]-7-oxo-4-oxa-1-azabicyclo-[3.2.0]hept-2-en-carbonsäure, ihren Na-Salz und ihrem p-nitrobenzylester

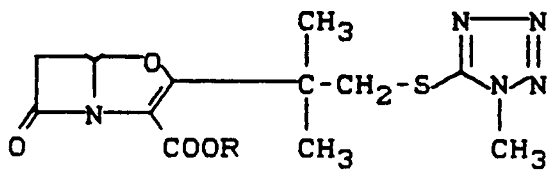

R = H, Na pNBz

Umwandlung einer Gruppe $R^3$; 2-(4-tert.-Butylthio-2-oxo-1-azetidinyl)-4,4-dimethyl-5-[(1-methyl-1,2,3,4-tetrazol-5-yl)-thiol-3-oxopentansäure-p-nitrobenzylester

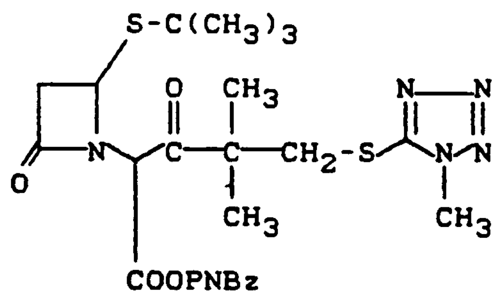

Ein Gemisch von 118 mg (0,25 mMol) 2-(4-tert.-Butylthio-2-oxo-1-azetidinyl)-5-chlor-4,4-dimethyl-3-oxopentansäure-p-nitrobenzylester und 82 mg (0.59 mMol) 1-Methyl-5-mercapto-1,2,3,4-tetrazol Na-Salz werden in 0.2 ml Dimethylformamid während 20 Stunden bei Raumtemperatur gerührt. Das Reaktionsgemisch wird direkt auf eine Chromatographie-Säule mit 6 g Kieselgel mit Toluol-Essigsäureethylester (19:1) chromatographiert, wobei 60 mg reine Titelverbindung erhalten werden als nichtkristalliner Festkörper.

Stufe $D_1$ un $D_2$: 3-[1,1-Dimethyl-2-((1-methyl-1,2,3,4-tetrazol-5-yl)-thio)-ethyl]-7-oxo-4-oxa-1-azabicyclo-[3.2.0]hept-2-en-2-carbonsäure-p-nitrobenzylester

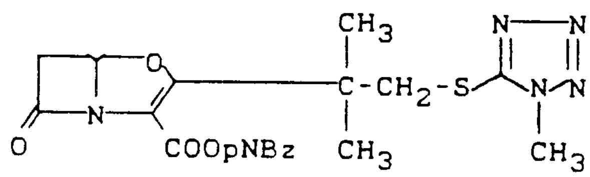

Ausgehend von 2-(4-tert.-Butylthio-2-oxo-1-azetidinyl)-4,4-dimethyl-5-[(1-methyl-1,2,3,4-tetrazol-5-yl)-thio]-3-oxo-pentansäure-p-nitrobenzylester wurde nach dem im Beispiel 1 gegebenen Verfahren über die Stufe $D_1$ und $D_2$ die Titelverbindung erhalten nach Chromatographie über Kieselgel mit Toluol-Essigsäureethylester (3:1). IR-Spektrum in $CH_2Cl_2$: 2930, 2860, 1810, 1720, 1590, 1525, 1350, 1320, 1175, 1120, 1030, 1015 $cm^{-1}$.

Stufe E: 3-[1,1-Dimethyl-2-((1-methyl-1,2,3,4-tetrazol-5-yl)-thio)-ethyl]-7-oxo-4-oxa-1-azabicyclo[3.2.0]hept-2-en-2-carbonsäure, Na-Salz

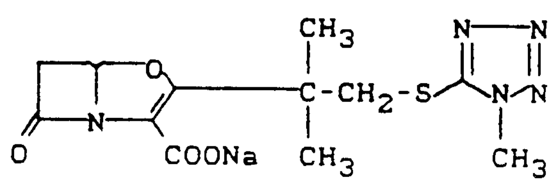

Ausgehend von dem entsprechenden p-Nitrobenzylester wurde nach dem im Beispiel 1 beschriebenen Verfahren über die Stufe E die Titelverbindung als weißer Festkörper (nach Lyophilisation erhalten, UV-Spektrum in $H_2O$: Starke Endabsorption, Schulter bei 270 nm ($\epsilon$ = 4000).

Beispiel 10

Herstellung von 3-[1-Methyl-1-(2-thienyl)-ethyl]-7-oxo-4-oxa-1-azabicyclo[3.2.0]hept-2-en-2-carbonsäure, ihrem Na-Salz und ihrem p-Nitrobenzylester

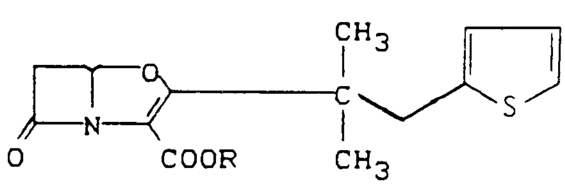

R = H, Na, pNBz

Ausgehend von 2-(4-tert.-butylthio-3,3-dimethyl-2-oxo-azetidinyl)-essigsäure-p-nitrobenzylester under 2-Methyl-2-thienylpropionsäurechlorid wurde nach dem im Beispiel 1 beschriebenen Verfahren über die Stufen C, $D_1$ und $D_2$ die Titelverbindung (p-Nitrobenzylester) erhalten als farbloser nichtkristalliner Festkörper. IR-Spektrum in $CH_2Cl_2$: 2930, 1800, 1720, 1605, 1530, 1350, 1080 $cm^{-1}$.

Stufe E: 3-[1-Methyl-1-(2-thienyl)-ethyl]-7-oxo-4-oxa-1-azabicyclo[3.2.0]hept-2-en-2-carbonsäure,Na-Salz

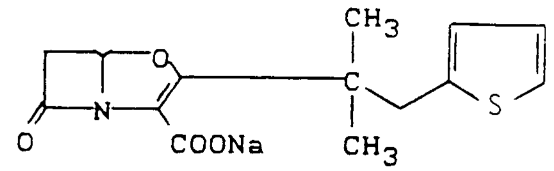

Ausgehend vom entsprechenden p-Nitrobenzylester wurde nach dem im Beispiel 1 beschriebenen Verfahren die reine Titelverbindung als weißer Festkörper erhalten. UV-Spektrum in $H_2O$: $\lambda_{max}$ = 270 nm ($\epsilon$ = 6500).

Beispiel 11

Herstellung von 2-(2-Acetoxy-1,1-dimethylethyl)-7-oxo-4-oxa-1-azabicyclo[3.2.0]hept-2-en-2-carbonsäure, ihrem Na-Salz und ihrem p-Nitrobenzylester

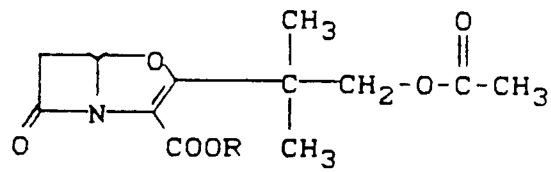

R = H, Na pNBz

Ausgehend von 2-(4-tert.-butylthio-2-oxoazetidinyl)-essigsäure-p-nitrobenzylester und ß-Acetoxypivalinsäurechlorid wurde nach dem im Beispiel 1 beschriebenen Verfahren die Titelverbindung (p-Nitrobenzylester) über die Stufen C, $D_1$ und $D_2$ als leicht gelblicher nichtkristalliner Festkörper erhalten. IR-Spektrum in $CH_2Cl_2$: 2950, 2850, 1810, 1740, 1720, 1590, 1550, 1340, 1080 $cm^{-1}$.

Stufe E: 2-(2-Acetoxy-1,1-dimethylethyl)-7-oxo-4-oxa-1-azabicyclo[3.2.0]hept-2-en-2-carbonsäure, Na-Salz

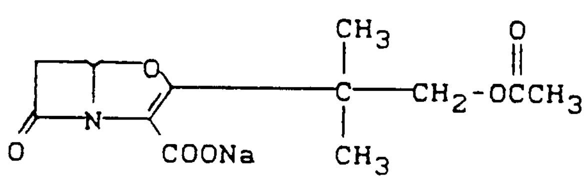

Ausgehend von dem entsprechenden p-Nitrobenzylester wurde nach dem in Beispiel 1 beschriebenen Verfahren über die Stufe E die Titelverbindung in 70 % Ausbeute als nichtkristalliner weißer Festkörper (Lyophilisat) erhalten.

UV-Spektrum in $H_2O$: $\lambda_{max}$ = 270 nm ($\epsilon$ = 6300). NMR-Spektrum in $D_2O$: $\delta$ = 1.25 (s, 3H), 1.26 (s, 3H) 2.06 (s, 3H), 3.40 (dd, J = 1 Hz, J = 17 Hz, 2 H) 3.72 (dd, J = 3 Hz, J = 17 Hz, 2 H) 4.23 (AB, J = 17 Hz, 2 H), 5.80 (dd, J = 1 Hz, J = 3 Hz, 1 H).

Beispiel 12

Herstellung von 3-tert.-Butyl-6-(1-hydroxyethyl)-7-oxo-4-oxa-1-azabicyclo[3.2.]hept-2-en-2-carbonsäure, ihrem Na-Salz und ihrem p-Nitrobenzylester

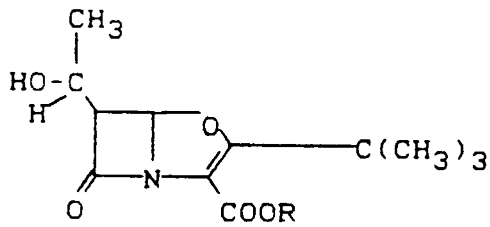

Stufe $A_2$: 4-Methylthio-3-[1-(p-nitrobenzyloxycarbonyloxy)-ethyl]-azetidin-2-on

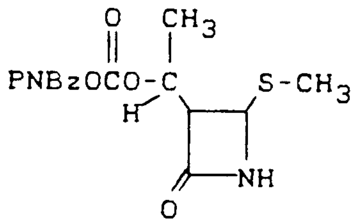

R = H, Na pNBz

Zu einer Lösung von 4.48 g (11.1 mMol) 4-(2-hydroxyethylsulfonyl)-3-[1-p-nitrobenzyloxycarbonyloxy)-ethyl]-azetidin-2-on in 11 ml Acetonitril und 11 ml $H_2O$ werden bei 0° C 1.17 g (16,7 mMol) Methylmercaptan-Na-Salz gegeben und das Gemisch während 20 Minuten bei 0° C gerührt. Die Reaktionsmischung wird verdünnt mit 100 ml Methylenchlorid und 25 ml $H_2O$ und nach Abtrennen der organischen Phase die wäßrige Phase dreimal mit je 25 ml Methylenchlorid extrahiert. Die vereinigten organischen Phasen werden über $MgSO_4$ getrocknet, filtriert und das Lösungsmittel im Vakuum entfernt, wobei 3.80 g Titelverbindung als leicht gelbliche nichtkristalline Verbindung erhalten werden. NMR-Spektrum in $CDCl_3$: $\delta$ = 1.45 (d, J = 7 Hz, 3 H), 2.12 (s, 3 H), 3.3. (dd, J = Hz, J = 2 Hz), 4.70 (d, J = 2 Hz, 1 H), 5.12 (m, 1 H), 5.20 (s, 2 H), 6,45 (breites s, 1 H), 7,50 (d, J = 8,5 Hz, 2 H), 820 (d, J = 8,5 Hz, 2 H).

Stufe B: 2-[4-Methylthio-3-(1-(p-nitrobenzyloxycarbonyl-oxy)-ethyl)-2-oxoazetidinyl]-essigsäure-p-nitrobenzylester

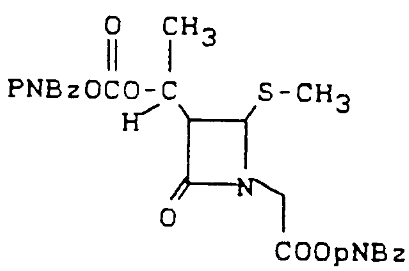

Zu einem Gemisch von 680 g (2 mMol) 4-Methylthio-3-[1-(p-nitrobenzyloxycarbonyloxy)-ethyl]-azetidin-2-on und 602 mg (2.2 mMol) Bromessigsäure-p-nitrobenzylester in 2 ml trockenem Tetrahydrofuran und 2 ml trockenem DMF werden unter Rühren bei -70° C 2.2 ml einer 1 M Lösung von Lithium-bis-(trimethylsilyl)amid innerhalb von 5 Minuten zugegeben. Das Reaktionsgemisch wird während 30 Minuten bei -70° C gerührt, mit 30 ml Essigsäureethylester und 70 ml Toluol verdünnt und zweimal mit verdünnter NaCl-Lösung gewaschen. Die organische Phase wird über $MgSO_4$ getrocknet, filtriert und das Lösungsmittel in Vakuum abgedampft. Der Rückstand wird über 65 g Kieselgel mit Toluol-Essigester 4:1 chromatographiert, wobei 520 mg eines gelben Öls erhalten werden. IR-Spektrum in $CH_2Cl_2$: 2920, 2850, 1765, 1750, 1605, 1520, 1355, 1345 $cm^{-1}$.

Stufe C: 4,4-Dimethyl-2-[4-Methylthio-3-1-(p-nitrobenzyloxy-carbonyloxy)-ethyl)-2-oxoazetidinyl]-3-oxopentansäure-p-nitrobenzylester

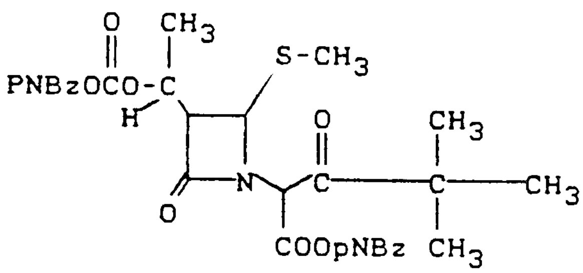

Zu einem Gemisch von 275 mg (0,515 mMol) 2-[4-Methylthio-3-(1-(p-nitrobenzyloxycarbonyloxy)-ethyl)-2-oxoazetidinyl]-essigsäure-p-nitrobenzylesterund 67 µl (0.55 mMol) Pivalinsäurechlorid in 6,7 ml trockenem Tetrahydrofuran werden bei -70° C unter Rühren 1,1 ml 1 M Lithium-bis(trimethylsilyl)-amid innerhalb von 5 Minuten zugegeben und das Reaktionsgemisch noch während 30 Minuten bei -70° C gerührt und dann mit 40 ml Toluol verdünnt und mit 30 ml 2 N HCl, dann zweimal mit je 40 ml NaCl-Lösung gewaschen. Die organische Phase wird über $MgSO_4$ getrocknet, filtriert und das Lösungsmittel im Vakuum entfernt. Der Rückstand wird über 10 g Kieselgel mit Toluol-Essigsäureethylester (9:1) chromatographiert, wobei 268 mg eines weißen nichtkristallinen Festkörpers erhalten werden. IR-Spektrum in $CH_2Cl_2$: 2930, 2850, 1775, 1760, 1720, 1610, 1535, 1350 $cm^{-1}$.

Stufe $D_1$: 2-[4-Chlor-3-(1-(p-nitrobenzyloxycarbonyloxy)-ethyl)-2-oxoazetidinyl]-4,4-dimethyl-3-oxo-pentansäure-p-nitrobenzylester

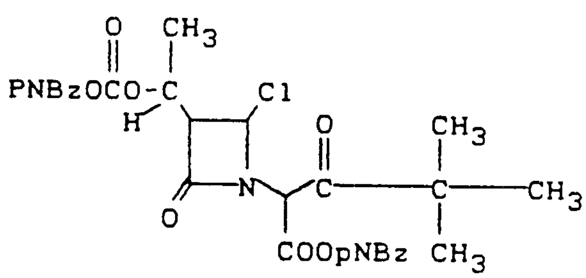

Eine Lösung von 244 mg (0.395 mMol) 4,4-Dimethyl-2-[4-Methylthio-3-(1-(p-nitrobenzyloxy-carbonyloxy)-ethyl)-2-oxoazetidinyl]-3-oxopentansäure-p-nitrobenzylester in 16 ml Methylenchlorid wird mit 660 µl einer 850 mg in 10 ml enthaltenden Lösung von Chlor in Tetrachlorkohlenstoff versetzt bei -60° C. Die schwach gelbe Lösung wird während 2 Stunden bei -60° C gerührt und das Lösungsmittel im Vakuum entfernt, wobei 236 mg eines farblosen nichtkristallinen Festkörpers erhalten werden. IR-Spektrum in $CH_2Cl_2$: 2930, 2850, 1795, 1765, 1725, 1620, 1530, 1355 $cm^{-1}$.

Stufe $D_2$: 3-tert.-Butyl-5-[1-(p-nitrobenzyloxycarbonyloxy)-ethyl]-7-oxo-4-oxa-1-azabicyclo[3.2.0]hept-2-en-carbonsäure-p-nitrobenzylester

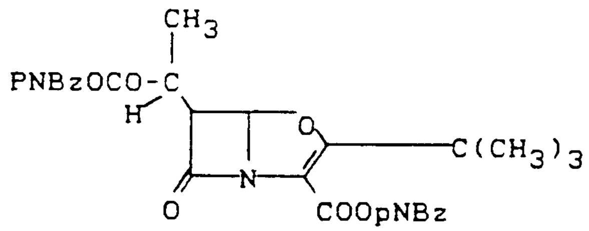

Eine Lösung von 214 mg 2-[4-Chlor-3-(1-(p-nitrobenzyloxycarbonyloxy)-ethyl)-2-oxoazetidinyl]-4,4-dimethyl-3-oxo-pentansäure-p-nitrobenzylester in 7 ml trockenem Tetrahydofuran wird bei -30° C unter Rühren mit 476 µl 0,75 M Kalium-tert.-butylat in tert.-Butanol versetzt und das Reaktionsgemisch anschließend während 30 Minuten bei -30° C gerührt. Die Reaktionslösung wird mit 40 ml Essigsäureethylester verdünnt und anschließend mit 40 ml verdünnter NaCl- und mit 40 ml gesättigter NaCl-Lösung gewaschen.

Trocknen der organischen Phase über $MgSO_4$, Filtration und Eindampfen des Lösungsmittels im Vakuum liefert 198 mg Rückstand, der über 6 g Kieselgel mit Toluol-Essigsäureethylester chromatographiert wird, wobei 183 mg eines farblosen nichtkristallinen Festkörpers (Titelverbindung) erhalten werden. IR-Spektrum in $CH_2Cl_2$: 3030, 2950, 1805, 1755, 1720, 1610, 1580, 1530, 1350, 1320, 1090 $cm^{-1}$.

Stufe E: Gleichzeitige Entfernung von zwei Schutzgruppen, 3-tert.-Butyl-6-(1-hydroxyethyl)-2-oxo-4-oxa-1-azabicyclo[3.2.0]hept-2-en-2-carbonsäure, Na-Salz

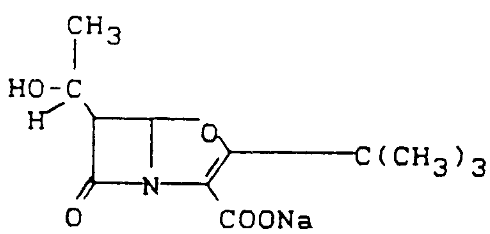

Zu einem vorhydrierten Gemisch von 84 mg Pd/C (10 %), 4,6 mg $NaHCO_3$ in 1 ml Essigsäureethylester und 0.7 ml $H_2O$ wird eine Lösung von 28 mg (0,05 mMol) 3-tert.-Butyl-6-[1-(p-nitrobenzylcarbonyloxy)-ethyl]-7-oxo-4-oxa-1-azabicyclo[3.2.0]hept-2-en-2-carbonsäure-p-nitrobenzylester in 1 ml Essigsäureethylester durch ein Septum mit einer Spritze bei 0° C zugegeben und das Gemisch bei 0° C während 40 Minuten hydriert, wobei 8.0 ml $H_2$ ausgenommen werden (theor. Wert ca. 8.8 ml $H_2$). Das Gemisch wird bei 0° C filtriert und die wäßrige Phase noch zweimal mit je 2 ml vorgekühltem Essigsäureethylester gewaschen und dann im Hochvakuum lyophilisiert, wobei 11 mg eines weißen nichtkristallinen Feststoffes (Titelverbindung) erhalten werden.
UV-Spektrum in $H_2O$: $\lambda_{max}$ = 278 nm ($\epsilon$ = 5500).

Beispiel 13

Herstellung von 3-tert.-Butyl-6-hydroxymethyl-7-oxo-4-oxa-1-azabicyclo[3.2.0]hept-2-en-2-carbonsäure und ihrem Na-Salz

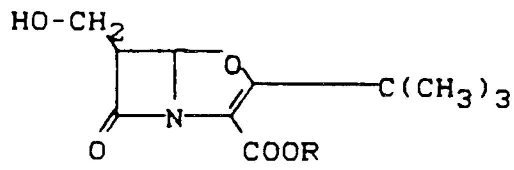

R = H, Na, p-NBz

Ausgehend von 4-(2-hydroxyethylsulfonyl)-3-(p-nitrobenzyloxy-carbonyloxymethyl)-azetidin-2-on wurde nach dem in Beispiel 12 beschriebenen Verfahren über die Stufe $A_2$, B, C, $D_1$, $D_2$ und E die Titelverbindung (Na-Salz) als weißer nichtkristalliner Festkörper (lyophilisiert) erhalten. UV-Spektrum in $H_2O$: $\lambda_{max}$ = 275 nm ($\epsilon$ = 5500).

Auf analoger Weise wurde das Kaliumsalz der 3-tert.-Butyl-6-hydroxymethyl-7-oxo-4-oxa-1-azabicyclo-[3.2.0]-hept-2-en-2-carbonsäure hergestellt.

Beispiel 14

Herstellung von 2-tert.-Butyl-6-ethyliden-1-oxapen-2-em-3-carbonsäure, ihrem Na-Salz und ihrem p-Nitrobenzylester

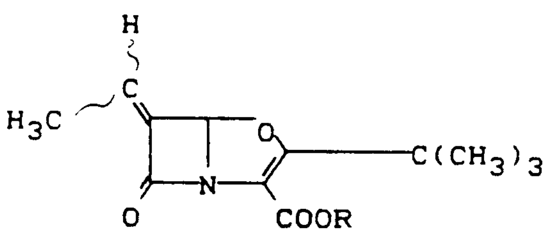

R = H, Na p-NBz

Ausgehend von 4-tert.-Butylthio-3-ethylidenazetidin-2-on wird über die Stufen B, C, D und $D_2$ unter Anwendung der im Beispiel 12 angegebenen Reagenzien und Reaktionsbedingungen die Titelverbindung (p-Nitrobenzylester) erhalten (3-tert.-Butyl-6-ethyliden-3-oxa-7-oxo-1-azabicyclo[3.2.0]hept-2-en-2-carbonsäure-p-nitrobenzylester).

IR Spektrum in $CH_2Cl_2$: 1800, 1720, 1585, 1525, 1345, 1310, 1165 $cm^{-1}$.

Stufe E: 3-tert.-Butyl-6-ethyliden-4-oxa-7-oxo-1-azabicyclo[3.2.0]hept-2-en-2-carbonsäure, Na-Salz

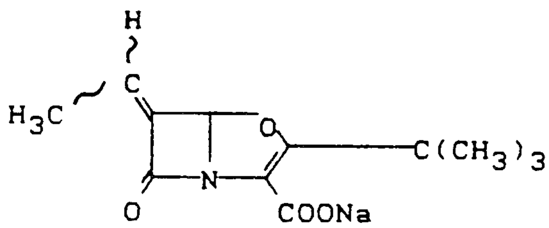

Ausgehend vom entsprechenden p-Nitrobenzylester wird unter Anwendung der im Beispiel 12 angegebenen Reaktionsbedingungen unter Verwendung von Pd auf $PbCO_3$ anstatt Pd auf C als Katalysator nach 4 Stunden Reaktionszeit die Titelverbindung erhalten (weißes Lyophilisat). UV-Spektrum in $H_2O$: $\lambda_{max}$ = 272 ($\epsilon$ = 5100).

Beispiel 15

Herstellung pharmazeutischer Zubereitungen

Eine Einheitsdosisform wird hergestellt, indem man 60 mg 3-tert.-Butyl-6-hydroxymethyl-7-oxo-4-oxa-1-azabicyclo[3.2.0]hept-2-en-2-carbonsäure, K-Salz mit 120 mg Ampicillin, 20 mg Lactose und 5 mg Magnesiumstearat vermischt und das 205 mg Gemisch in eine Nr. 3 Gelatinekapsel gibt. Ähnliches kann man, wenn man mehr aktive Bestandteile und weniger Lactose verwendet, andere Dosisformen herstellen und in Nr. 3 Gelatinekapseln füllen; und sollte es erforderlich sein, mehr als 205 mg Bestandteile zusammen zu vermischen, können größere Kapseln, wie auch komprimierte Tabletten und Pillen, ebenfalls hergestellt werden. Die folgenden Beispiele erläutern die Herstellung pharmazeutischer Zubereitungen.

Tabelle

| | mg |
|---|---|
| 3-tert.-Butyl-6-hydroxymethyl-7-oxo-4-oxa-1-azabicyclo[3.2.0]hept-2-en-2-carbonsäure, K-Salz | 60 |
| Ampicillin | 120 |
| Maisstärke V.S.P. | 6 |
| Magnesiumstearat | 232 |
| Dicalciumphosphat | 192 |
| Lactose, V.S.P. | 190 |

Die aktiven Bestandteile werden mit dem Dicalciumphosphat, Lactose und etwa der Hälfte der Maisstärke vermischt. Das Gemisch wird dann mit 6 mg Maisstärke granuliert und grob gesiebt. Es wird im Hochvakuum getrocknet und erneut durch Siebe mit lichten Maschenweiten von 1.00 mm (Nr. 16 screens) gesiebt. Der Rest der Maisstärke und das Magnesiumstearat werden zugegeben und das Gemisch wird zu Tabletten, die je 800 mg wiegen und einen Durchmesser von etwa 1,27 cm (0,5 in.) besitzen, verpreßt.

Parenterale Lösung

| Ampulle | |
|---|---|
| 3-tert.-Butyl-6-hydroxymethyl-7-oxo-4-oxa-1-azabicyclo[3.2.0]-hepten-2-carbonsäure K-Salz | 100 mg |
| Ampicillin | 500 mg |
| steriles Wasser (wird aus einer separaten Ampulle mit der Spritze unmittelbar vor der Verwendung zugegeben) | 2 ml |

| Ophthalmische Lösung | |
|---|---|
| 3-tert.-Butyl-6-hydroxymethyl-7-oxo-4-oxa-1-azabicyclo[3.2.0]-hepten-2-carbonsäure K-Salz | 20 mg |
| Ampicillin | 100 mg |
| Hydroxypropylmethylcellulose | 5 mg |
| steriles Wasser bis zu (wird aus einer separaten Ampulle mit der Spritze unmittelbar vor der Verwendung zugegeben) | 1 mg |

| Optische Lösung | |
|---|---|
| 2-tert.-Butyl-6-hydroxymethyl-7-oxo-4-oxa-1-azabicyclo[3.2.0]-hepten-2-carbonsäure K-Salz | 20 mg |
| Ampicillin | 100 mg |
| Benzalkoniumchlorid | 0,1 mg |
| steriles Wasser bis zu (wird aus einer separaten Ampulle mit der Spritze unmittelbar vor der Verwendung zugegeben) | 1 ml |

| Topische Creme bzw. Salbe | |
|---|---|
| 3-tert.-Butyl-6-hydroxymethyl-7-oxo-4-oxa-1-azabicyclo[3.2.0]hepten-2-carbonsäure K-Salz | 20 mg |
| Ampicillin | 100 mg |
| Polyethylenglykol 4000 V.S.P. | 400 mg |
| Polyethylenglykol 400 V.S.P. | 0,1 g |

**Patentansprüche**

**1.** Verwendung von Verbindungen der Strukturformeln

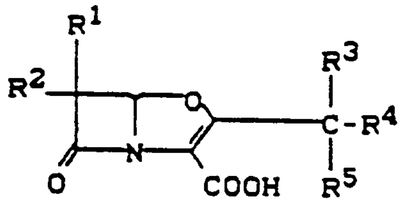

bzw.

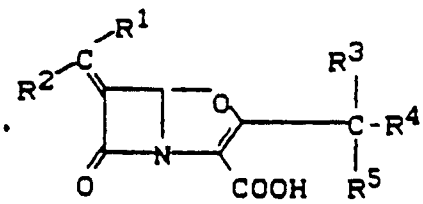

ihre pharmazeutisch annehmbaren Salze. Ester und Amidderivate, worin $R^1$ und $R^2$ unabhängig voneinander Wasserstoff oder Ober Kohlenstoff-Kohlenstoff-Einfachbindungen mit dem übrigen Molekülteil gebundene pharmazeutisch annehmbare Gruppen bedeuten, die ausgewählt werden aus substituiertem oder unsubstitituiertem Alkyl, Alkenyl, Alkinyl, Cycloalkyl, Alkylcycloalkyl, Alkylcycloalkenyl, Cycloalkylalkyl, Alkenylcycloalkyl, Cycloalkenylalkyl, Aryl, Aralkyl, Aralkenyl, Aralkinyl, Carboxy oder Cyano, worin die vorhergehenden Alkyl-, Alkenyl- oder Alkinyl-Molekülteile 1 bis 6 Kohlenstoffatome, die Cycloalkyl- oder die Cycloalkenyl-Molekülteile 3 bis 6 und die Aryl-Molekülteile 6 bis 10 Kohlenstoffatome enthatten, Heteroaryl, Heteroaralkyl, Heteroaralkenyl, Heteroaralkinyl, Alkylheteroaryl, Heterocyclyl, Heterocyclylalkyl, Heterocyclylalkenyl, Heterocyclylalkinyl, Alkylheterocyclyl, worin die vorhergehenden Alkyl-, Alkenyl- oder die Alkinyl-Molekülteile 1 bis 6 Kohlenstoffatome enthalten und der heteroaromatische oder heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und wobei die Substituenten der oben augeführten Gruppen sein können: Geschütztes oder ungeschütztes Hydroxy, Hydroxyalkyloxy, Aminoalkyloxy, Amidinoalkyloxy, Alkyloxy, Acyloxy, Aryloxy, Heteroaryloxy, Heterocyclyloxy, Carbamoyl, Carbamoyloxy, Thiocarbamoyl, Thiocarbamoyloxy, Alkylcarbamoyloxy, Alkylthiocarbamoyloxy, Mercapto, Alkylthio, Hydroxyalkythio, Aminoalkylthio, Amidinoalkythio, Acylthio, Arylthio, Alkylheteroarylthio, Hydroxyalkylheteroarylthio, Heterocyclylthio, Carbamoylthio, Alkylcarbamoylthio, Thiocarbamoylthio, Alkylthiocarbamoylthio, geschütztes oder ungeschütztes Amino oder Monoalkylamino, Dialkylamino, Oxo, geschütztes oder ungeschüztes Oximino oder Alkylimino, Tetraalkylammonium, Cycloalkylamino, Arylamino, Heteroarylamino, Heterocyclylamino, Acylamino, Amidino, Alkylamidino, Guanidino, Alkylguanidino, Carbamoylamino, Alkylcarbamoylamino, Thiocarbamoylamino, Alkylthiocarbamoylamino, Nitro, Chlor, Brom, Fluor, Iod, Azido, Cyano, Alkylsulfinyl, Alkylsulfonyl, Sulfonamido, Sulfamoyloxy, Alkylsulfonyloxy oder geschütztes oder ungeschütztes Sulfo, Sulfoxy oder Carboxy, wobei die Substituenten unabhängig voneinander ein- oder mehrfach auftreten und ihr Alkyl-Molekülteil 1 bis 6 Kohlenstoffatome, ihr Aryl-Molekülteil 6 bis 10 Kohlenstoffatome enthält, und wobei der heteroaromatische oder heterocyclische Molekülteil mono- oder bicyclisch ist und 3 bis 10 Ringatome enthält, wovon eines oder mehrere ausgewählt werden aus der Gruppe: Sauerstoff, Schwefel und Stickstoff, und worin daß $R^3$, $R^4$ und $R^5$ unabhängig voneinander ausgewählt werden aus den vorhergehend genannten, über Kohlenstoff-Kohlenstoff-Einfachbindungen an den übrigen Molekülteil gebundenen pharmazeutisch annehmbaren Gruppen, zur Herstellung $\beta$-Lactamasehemmender der Arzneimittel.

**2.** Verwendung von Verbindung nach Anspruch 1, dadurch gekennzeichnet, dass $R^3$, $R^4$ und $R^5$ die oben angegebene Bedeutungen haben und $R^1$ und $R^2$ unabhängig voneinander Wasserstoff, Alkyl, geschütztes oder ungeschütztes Hydroxyalkyl oder geschütztes oder ungeschütztes Dihydroxyalkyl mit jeweils 1 bis 6 Kohlenstoffatomen bedeuten, zur Herstellung $\beta$-Lactamase-hemmender Arzneimittel.

**3.** Verwendung von Verbindungen nach Anspruch 1, dadurch gekennzeichnet, dass $R^1$ und $R^2$ die darin angegebenen Bedeutungen haben $R^3$ und $R^4$ Methyl bedeuten und $R^5$ ausgewählt wird unter den Gruppen:

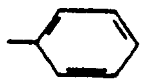
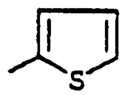
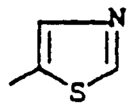

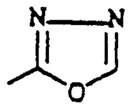
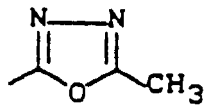
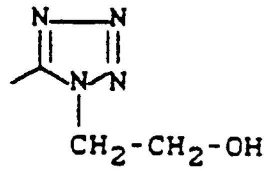

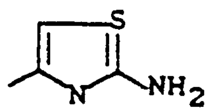
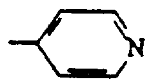
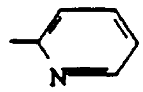

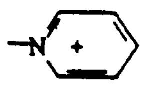
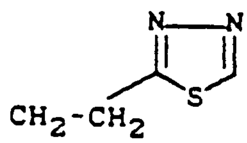
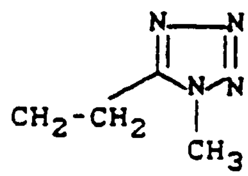

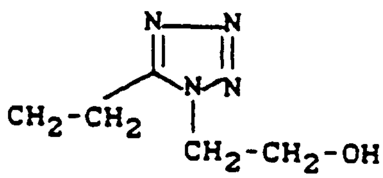
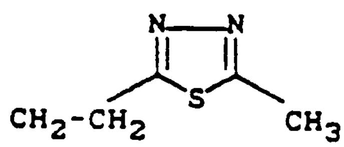

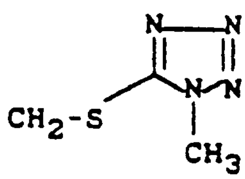
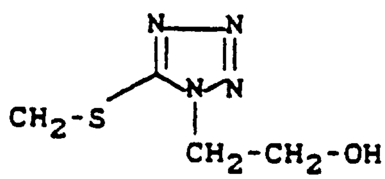
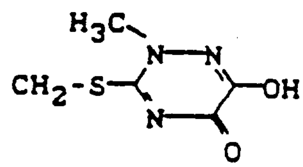

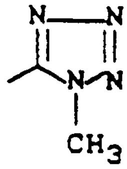 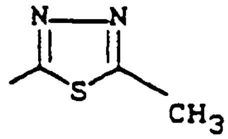 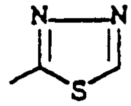

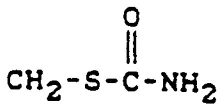 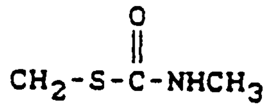 $CH_2-S-CH_3$

$CH_2-S-CH_2-CH_2-NH_2$ 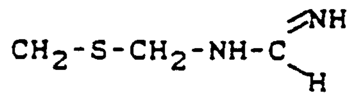

$CH_2-NH_2$ 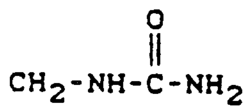 $CH_2-NH-CHO$

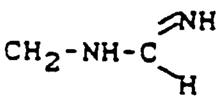 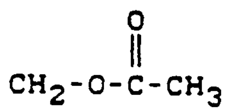 $CH_2-O-CHO$

$CH_2-Cl$ 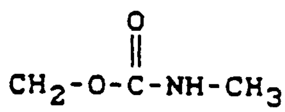 $CH_2-N_3$

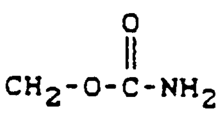 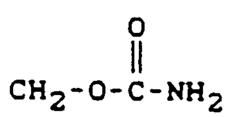 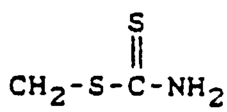

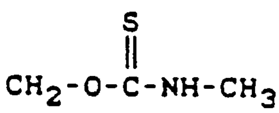 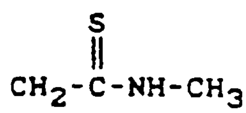 $CH_2-CH_2-NH_2$

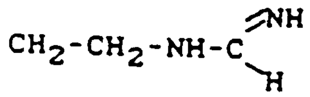

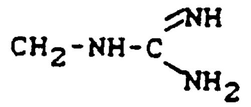

$CH_2-CH_2-CH_2-NH_2$

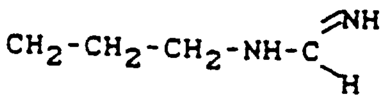

$CH_2-CH_2-COOH$

$CH_2-COOH$

$COOH$

$CH_2-CH_2-CH_2-COOH$

$CH_2-OH$

$CH_2-CH_2-CH_2-OH$

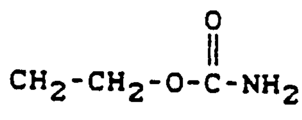

$C\equiv N$

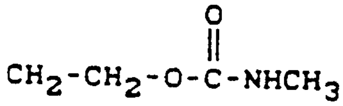

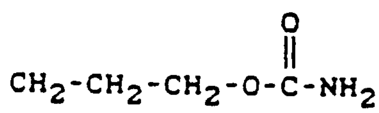

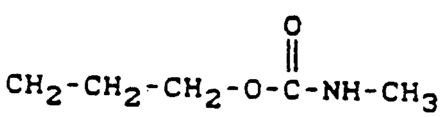

$CH_2-CH_2-CH_2-NH-CHO$

zur Herstellung β-Lactamase-hemmender Arzneimittel.

## Claims

1. Use of compounds of the structural formulae

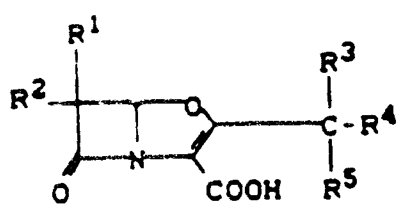

or

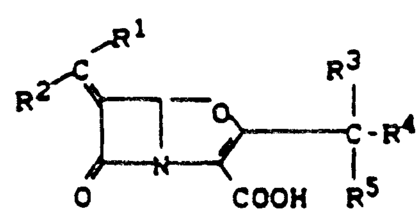

their pharmaceutically acceptable salts, esters and amide derivatives, in which $R^1$ and $R^2$ independently of one another denote hydrogen or pharmaceutically acceptable groups bonded via carbon-carbon single bonds with the other part of the molecule, which are selected from substituted or unsubstituted alkyl, alkenyl, alkynyl, cycloalkyl, alkylcycloalkyl, alkylcycloalkenyl, cycloalkylalkyl, alkenylcycloalkyl, cycloalkenylalkyl, aryl, aralkyl, aralkenyl, aralkynyl, carboxyl or cyano, in which the preceding alkyl, alkenyl or alkynyl parts of the molecule contain 1 to 6 carbon atoms, the cycloalkyl or the cycloalkenyl parts of the molecule contain 3 to 6 and the aryl parts of the molecule contain 6 to 10 carbon atoms, heteroaryl, heteroaralkyl, heteroaralkenyl, heteroaralkynyl, alkylheteroaryl, heterocyclyl, heterocyclylalkyl, heterocyclylalkenyl, heterocyclylalkynyl, alkylheterocyclyl, in which the preceding alkyl, alkenyl or the alkynyl parts of the molecule contain 1 to 6 carbon atoms and the heteroaromatic or heterocyclic part of the molecule is mono- or bicyclic and contains 3 to 10 ring atoms, of which one or more are selected from the group comprising: oxygen, sulphur and nitrogen, and where the substituents of the abovementioned groups may be: protected or unprotected hydroxyl, hydroxyalkyloxy, aminoalkyloxy, amidinoalkyloxy, alkyloxy, acyloxy, aryloxy, heteroaryloxy, heterocyclyloxy, carbamoyl, carbmoyloxy, thiocarbamoyl, thiocarbamoyloxy, alkylcarbamoyloxy, alkylthiocarbamoyloxy, mercapto, alklythio,

hydroxyalkylthio, aminoalkythio, amidinoalkylthio, acylthio, arylthio, alkylheteroarylthio, hydroxyalkylheteroarylthio, heterocyclylthio, carbamoylthio, alkylcarbmoylthio, thiocarbamoylthio, alkylthiocarbamoylthio, protected or unprotected amino or monoalkylamino, dialkylamino, oxo, protected or unprotected oximino or alkylimino, tetraalkylammonium, cycloalkyamino, arylamino, heteroarylamino, heterocyclylamino, acylamino, amidino, alklamidino, guanidino, alkylguanidino, carbamoylamino, alkylcarbamoylamino, thiocarbamoylamino, alkylthiocarbamoylamino, nitro, chlorine, bromine, fluorine, iodine, azido, cyano, alkylsulphinyl, alkylsulphonyl, sulphonamido, sulphamoyloxy, alkylsulphonyloxy or protected or unprotected sulpho, sulphoxy or carboxyl, where the substituents independently of one another occur one or more times and the alkyl part of the molecule thereof contains 1 to 6 carbon atoms, the aryl part of the molecule thereof contains 6 to 10 carbon atoms, and where the heteroaromatic or heterocyclic part of the molecule is mono- or bicyclic and contains 3 to 10 ring atoms, of which one or more are selected from the group comprising: oxygen, sulphur and nitrogen, and in which $R^3$, $R^4$ and $R^5$ independently of one another are selected from the previously mentioned pharmaceutically acceptable groups bonded to the other part of the molecule by carbon-carbon single bonds, for the manufacture of $\beta$-lactamase-inhibiting medicaments.

**2.** Use of compounds according to Claim 1, characterized in that $R^3$, $R^4$ and $R^5$ have the abovementioned meanings and $R^1$ and $R^2$ independently of one another denote hydrogen, alkyl, protected or unprotected hydroxyalkyl or protected or unprotected dihydroxyalkyl each having 1 to 6 carbon atoms, for the manufacture of $\beta$-lactamase-inhibiting medicaments.

**3.** Use of compounds according to Claim 1, characterized in that $R^1$ and $R^2$ have the meanings indicated therein, $R^3$ and $R^4$ denote methyl and $R^5$ is selected from amongst the groups comprising:

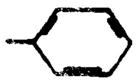
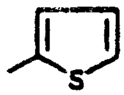
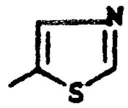
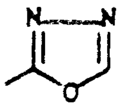
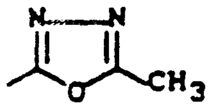
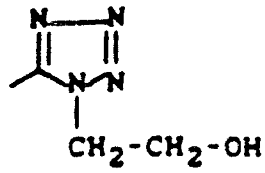
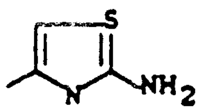
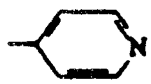
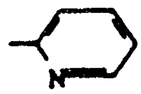
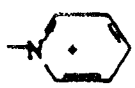
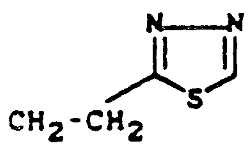
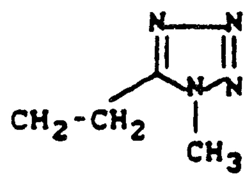
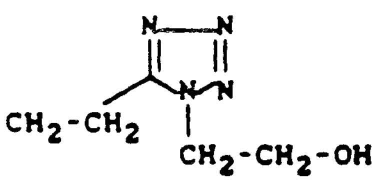
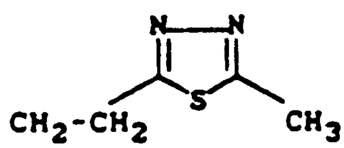
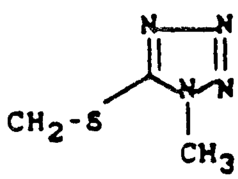
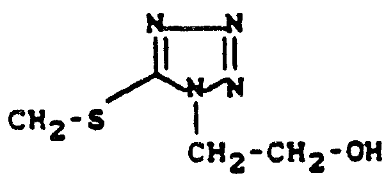
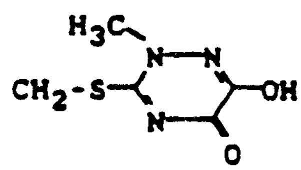
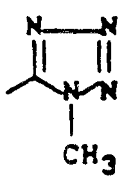
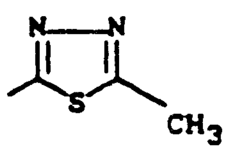
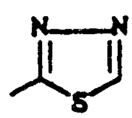

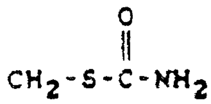
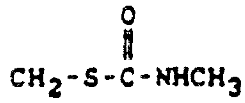
$CH_2-S-CH_3$

$CH_2-S-CH_2-CH_2-NH_2$
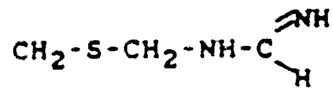

$CH_2-NH_2$
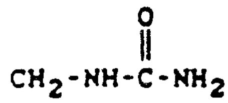
$CH_2-NH-CHO$

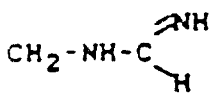
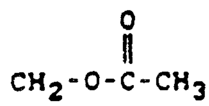
$CH_2-O-CHO$

$CH_2-Cl$
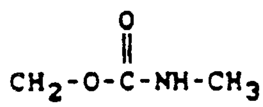
$CH_2-N_3$

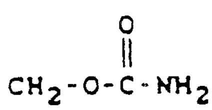
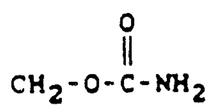
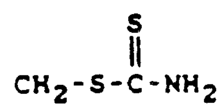

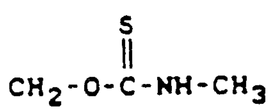
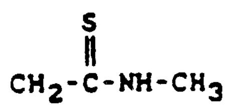
$CH_2-CH_2-NH_2$

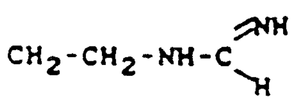
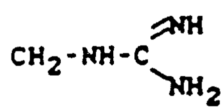
$CH_2-CH_2-CH_2-NH_2$

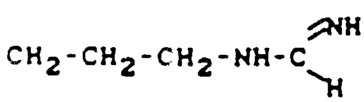
$CH_2-CH_2-COOH$
$CH_2-COOH$

$COOH$
$CH_2-CH_2-CH_2-COOH$
$CH_2-OH$

$CH_2-CH_2-CH_2-OH$
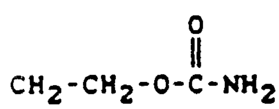
$C\equiv N$

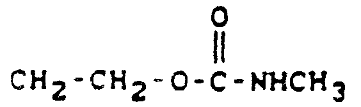
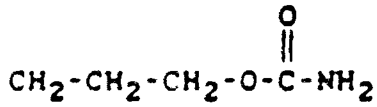

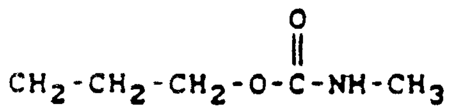
$CH_2-CH_2-CH_2-NH-CHO$

for the manufacture of $\beta$-lactamase-inhibiting medicaments.

**Revendications**

1. Utilisation de compose's répondant aux formules de structure

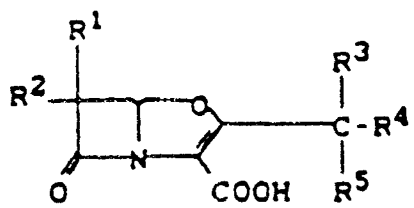, respectivement 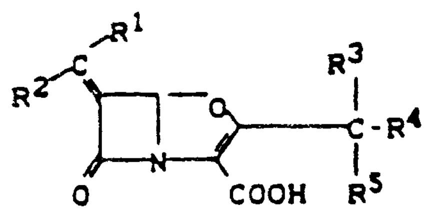

leurs sels, esters et dérivés amides pharmaceutiquement acceptables, dans lesquelles $R^1$ et $R^2$ représentent indépendamment l'un de l'autre, un atome d'hydrogène ou des groupes pharmaceutiquement acceptables liés au reste de la molécule par des simples liaisons carbone-carbone, qui sont choisis parmi un groupe alkyle substitué ou non-substitué, un groupe alcényle, un groupe alcinyle, un groupe cycloalkyle, un groupe alkylcycloalkyle, un groupe alkylcycloalcényle, un groupe cycloalkylalkyle, un groupe alcénylcycloalkyle, un groupe cycloalcénylalkyle, un groupe aryle, un groupe arylalkyle, un groupe arylalcényle, un groupe arylalcinyle, un groupe carboxyle ou un groupe cyano, où les fractions moléculaires d'alkyle, d'alcényle ou d'alcinyle précédentes contiennent de 1 à 6 atomes de carbone, les fractions moléculaires de cycloalkyle ou de cyclalcényle de 3 à 6 atomes de carbone et les fractions moléculaires d'aryle de 6 à 10 atomes de carbone, un groupe hétéroaryle, un groupe hétéroarylalkyle, un groupe hétéroarylalcényle, un groupe hétéroarylalcinyle, un groupe alkylhétéroaryle, un groupe hétérocyclyle, un groupe hétérocyclylalkyle, un groupe hétérocyclylalcényle, un groupe hétérocyclylalcinyle, un groupe àlkylhétérocyclyle, où les fractions moléculaires d'alkyle, d'alcényle ou d'alcinyle précédentes contiennent de 1 à 6 atomes de carbone et où la fraction moléculaire hétéroaromatique ou hétérocyclique est mono- ou bicyclique et contient de 3 à 10 atomes cycliques, dont un ou plusieurs sont choisis parmi le groupe formé par l'oxygène, le soufre et l'azote, et où les substituants des groupes énumérés ci-dessus peuvent représenter un groupe hydroxyle protégé ou non-protégé, un groupe hydroxyalkyloxy, un groupe aminoalkyloxy, un groupe amidinoalkyloxy, un groupe alkyloxy, un groupe acyloxy, un groupe aryloxy, un groupe hétéroaryloxy, un groupe hétérocyclyloxy, un groupe carbamoyle, un groupe carbamoyloxy, un groupe thiocarbamoyle, un groupe thiocarbamoyloxy, un groupe alkylcarbamoyloxy, un groupe alkylthiocarbamoyloxy, un groupe mercapto, un groupe alkylthio, un groupe hydroxyalkylhétéroarylthio, un groupe hétérocyclylthio, un groupe carbamoylthio, un groupe alkylcarbamoylthio, un groupe thiocarbamoylthio, un groupe alkylthiocarbamoylthio, un groupe amino ou monoalkylamino protégé ou non-protégé, un groupe dialkylamino, un groupe oxo, un groupe oximino ou alkylimino protégé ou non-protégé, un groupe tétraalkylammonium, un groupe cycloalkylamino, un groupe arylamino, un groupe hétéroarylamino, un groupe hétérocyclomino, un groupe amidino, un groupe alkylamidino, un groupe guanidino, un groupe alkylguanidino, un groupe carbamoylamino, un groupe alkylcarbamoylamino, un groupe thiocarbamoylamino, un groupe alkylthiocarbamoylamino, un groupe nitro, un atome de chlore, un atome de brome, un atome de fluor, un atome d'iode, un groupe azido, un groupe cyano, un groupe alkylsulfinyle, un groupe alkylsulfonyle, un groupe sulfonamido, un groupe sulfamoyloxy, un groupe alkylsulfonyloxy ou un groupe sulfo, sulfoxy ou carboxy protégé ou non-protégé, où des substituants sont présents, indépendamment l'un

de l'autre, une ou plusieurs fois et où leur traction moléculaire alkyle contient de 1 à 6 atomes de carbone, leur fraction moléculaire d'aryle de 6 à 10 atomes de carbone et où leur fraction moléculaire hétéroaromatique ou hétérocyclique est mono-ou bicyclique et contient de 3 à 10 atomes cycliques, dont un ou plusieurs sont choisis parmi le groupe formé par l'oxygène, le soufre et l'azote et dans laquelle $R^3$, $R^4$ et $R^5$ sont choisis, indépendamment l'un de l'autre, parmi les groupes pharmaceutiquement acceptables énumérés ci-dessus et liés au reste de la molécule par de simples liaisons carbone-carbone, pour la préparation de médicaments inhibant la $\beta$-lactamase.

**2.** Utilisation de composés selon la revendication 1, caractérisée en ce que $R^3$, $R^4$ et $R^5$ présentent la signification indiquée ci-dessus et $R^1$ et $R^2$ représentent indépendamment l'un de l'autre, un atome d'hydrogène, un groupe alkyle, un groupe hydroxyalkyle protégé ou non-protégé ou un groupe dihydroxyalkyle protégé ou non-protégé avec respectivement de 1 à 6 atomes de carbone, pour la préparation de médicaments inhibant la $\beta$-lactamase.

**3.** Utilisation de composés selon la revendication 1, caractérisée en ce que $R^1$ et $R^2$ présentent la signification indiquée dans celle-ci et que $R^3$ et $R^4$ représentent un groupe méthyle et que $R^5$ est choisi parmi les groupes :

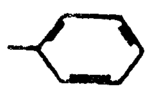 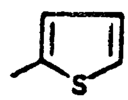 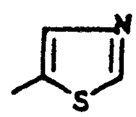

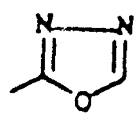 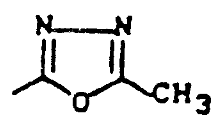 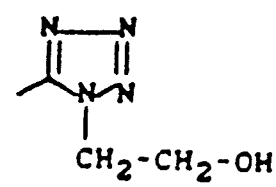

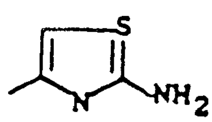 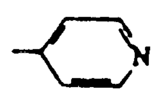 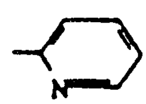

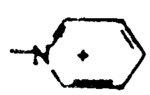 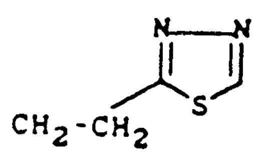 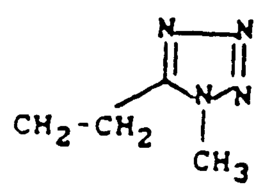

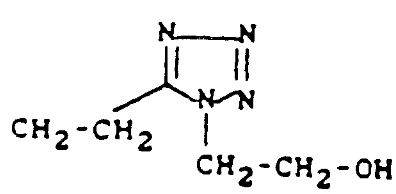 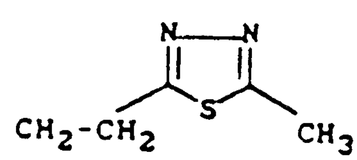

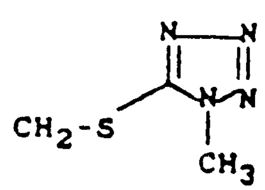 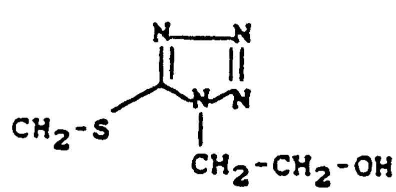 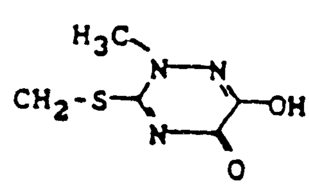

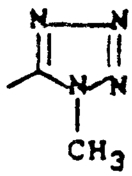
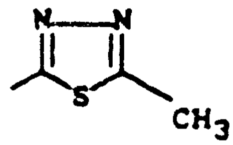
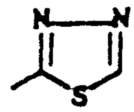

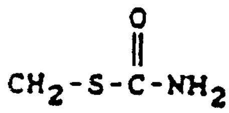
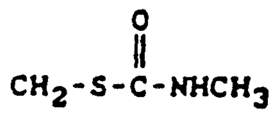
$CH_2-S-CH_3$

$CH_2-S-CH_2-CH_2-NH_2$
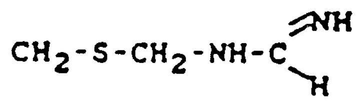

$CH_2-NH_2$
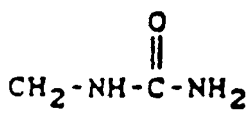
$CH_2-NH-CHO$

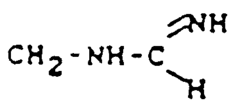
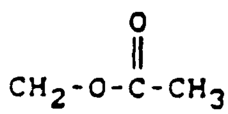
$CH_2-O-CHO$

$CH_2-Cl$
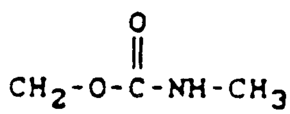
$CH_2-N_3$

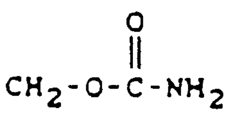
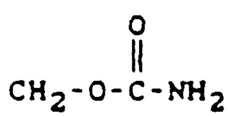
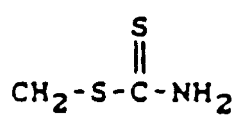

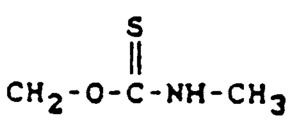
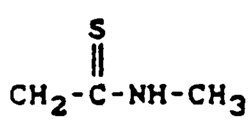
$CH_2-CH_2-NH_2$

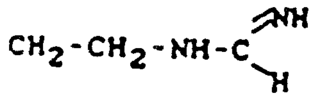

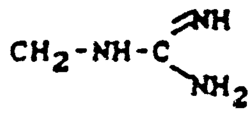

$CH_2-CH_2-CH_2-NH_2$

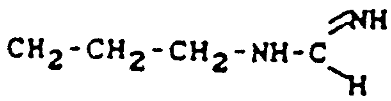

$CH_2-CH_2-COOH$

$CH_2-COOH$

$COOH$

$CH_2-CH_2-CH_2-COOH$

$CH_2-OH$

$CH_2-CH_2-CH_2-OH$

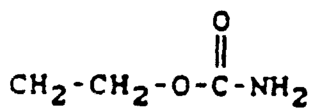

$C\equiv N$

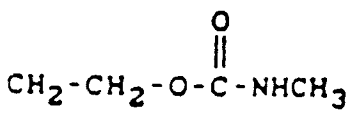

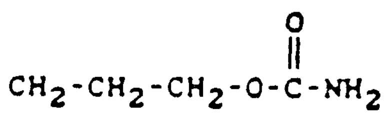

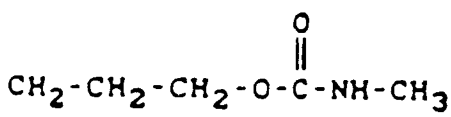

$CH_2-CH_2-CH_2-NH-CHO$

pour la préparation de médicaments inhibant la β-lactamase.